# EUROPEAN PATENT SPECIFICATION

(11) **EP 3 984 536 B1**
(45) Date of publication and mention of the grant of the patent: **25.06.2025**
(21) Application number: 20306238.5
(22) Date of filing: 19.10.2020
(51) Int. Cl.: A61K 31/4422, A61K 31/502, A61P 31/18

(54) **INHIBITION OF THE HIV-1 REPLICATION BY COMPOUNDS DIRECTED AGAINST A NEW TARGET OF THE VIRAL CYCLE**
HEMMUNG DER HIV-1-REPLIKATION DURCH VERBINDUNGEN, DIE GEGEN EIN NEUES TARGET DES VIRALEN ZYKLUS GERICHTET SIND
INHIBITION DE LA RÉPLICATION DU VIH-1 PAR DES COMPOSÉS DIRIGÉS CONTRE UNE NOUVELLE CIBLE DU CYCLE VIRAL

(43) Date of publication of application: 20.04.2022
(73) Proprietor: Centre national de la recherche scientifique, 75016 Paris (FR); Université Paris-Saclay, 91190 Gif-sur-Yvette (FR)
(72) Inventor: MIRANDE, Marc, 92350 LE PLESSIS ROBINSON (FR); COMISSO, Martine, 91140 VILLEBON SUR YVETTE (FR); DELELIS, Olivier, 78220 VIROFLAY (FR); SUBRA, Frédéric, 60500 CHANTILLY (FR)
(74) Representative: Novagraaf Technologies

(56) References cited:
- WO-A1-03/037349
- WO-A1-2005/113508
- WO-A1-2012/011917

## Description

The present invention concerns inhibitors of the initiation step of the reverse transcription of RNA of a type 1 human immunodeficiency virus (HIV-1).

The present invention also concerns inhibitors for use in the treatment of the acquired immune deficiency syndrome.

The present invention also concerns compound of formula (I) or (II) for use in the treatment of the acquired immune deficiency syndrome and a method of therapeutic treatment comprising the administration of such an inhibitor or compound of formula (I) or (II).

### STATE OF THE ART

WO2012/011917 relates to compositions comprising rifalazil and methods for treating a bacterial infection.

WO03/037349 relates to the use of type 4 phosphodiesterase inhibitors (PDE IV inhibitors) to treat diseases and to combinations of PDE IV inhibitors with other drugs.

WO2005/113508 describes a crystal of 6-(3-chloro-2-fluorobenzyl)-1-[(S)-1-hydroxymethyl-2-methylpropyl]-7-methoxy-4-oxo-1,4-dihydroquinoline-3-carboxylic acid.

Human immunodeficiency virus (HIV) is the etiological agent responsible for acquired immune deficiency syndrome (AIDS), a fatal disease characterized by destruction of the immune system and the degeneration of the central and peripheral nervous system.

Currently, there are several antiviral drugs available anti-HIV. These drugs can be divided into several classes based on the viral protein they target and their mode of action: nucleoside and nucleotide reverse transcriptase inhibitors, non-nucleoside reverse transcriptase inhibitors, protease inhibitors, fusion inhibitors, integrase inhibitors and CCR5 receptor antagonists. Treatments against HIV are usually combinations of several antiviral drugs belonging to different classes (tritherapies).

However, not all patients are responsive to these treatments. In most cases, treatment failure is caused by the emergence of viral resistance, due to the rapid turnover of HIV, in particular of HIV-1, during the course of infection combined with a high viral mutation rate. Also, most of the drugs have known side effects.

Thus, there is still a need for the development of further new anti-HIV drugs, particularly the development of anti-HIV drugs having a new mechanism.

### AIMS OF THE INVENTION

One aim of the present invention is to solve the technical problem of providing an improved treatment against HIV, in particular HIV-1, that could alleviate the above limitations.

In particular, the present invention aims to solve the technical problem of providing anti-HIV drugs having a new mechanism, in particular providing anti-HIV-1 drugs.

### DETAILLED DESCRIPTION OF THE INVENTION

The present invention concerns a compound inhibitor of the initiation step of the reverse transcription of RNA of a type 1 human immunodeficiency virus (HIV-1) for use in a method for treating a type 1 human immunodeficiency virus (HIV-1).

Preferably, the compound inhibitor of the initiation step of the reverse transcription of RNA of a type 1 human immunodeficiency virus inhibits the interaction between an integrase of the type 1 human immunodeficiency virus and a mitochondrial lysyl-tRNA synthetase of a cell infected by the type 1 human immunodeficiency virus.

Type 1 human immunodeficiency virus (HIV-1) is a retrovirus whose genome made up of two single-stranded RNA molecules. The reverse transcriptase encoded by HIV-1 uses the tRNA₃^{Lys} of the host cell to initiate replication of its RNA into proviral DNA. tRNA₃^{Lys} is encapsulated into virions during assembly; host cellular mitochondrial lysyl-tRNA synthetase (LysRS), and in particular its mature form human mitochondrial lysyl-tRNA synthetase (mLysRS), is involved in this mechanism and serves as a co-transporter for tRNA3^{Lys}.

The protein-protein interactions involved in the formation of the tRNA₃^{Lys} encapsulating complex can be modelled as the interactions between the Pol domain of the precursor GagPol, in particular the transframe TF and integrase IN subdomains of the Pol domain, and the catalytic domain of mLysRS (Kobbi et al. J. Mol. Biol. 2011, 410:875).

Surprisingly, it was discovered by present inventors that it is possible to inhibit the initiation step of the reverse transcription of RNA of a type 1 human immunodeficiency virus by inhibiting the interaction between the precursor GagPol of a type 1 human immunodeficiency virus (UniProtKB - P12497 (POL_HV1N5)) and catalytic domain of mLysRS (residues 259 to 618 from H6-mLysRS-HA sequence) of the host cell, which inhibits the assembly of GagPol-tRNA₃^{LYS}-mLysRS complex. Because the primer RNA is not packaged into the virus, this prohibits the initiation step of the reverse transcription of RNA of a HIV-1.

Sequence of HIV-1 virus is for instance the plasmid sequence pNL4-3 (GenBank: AF324493.2).

Inhibitors according to the invention are able to inhibit the initiation step of the reverse transcription of RNA of a type 1 human immunodeficiency virus.

Inhibitors according to the invention have anti-viral activity without significant cellular toxicity.

The invention also concerns a compound of formula (I) for use as an inhibitor of the initiation step of the reverse transcription of RNA of a type 1 human immunodeficiency virus (HIV-1), preferably for use in a method of treatment of type 1 human immunodeficiency virus (HIV-1), wherein said formula (I) is as follows: 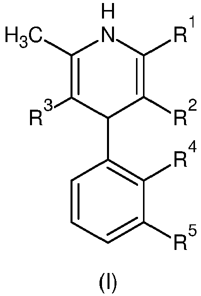
wherein R¹ is selected form the group consisting of:
   - a hydrogen atom,
   - an amino group NH₂, and
   - a C1-C10 alkyl group, linear or cyclic or branched, saturated or unsaturated, optionally comprising an heteroatom chosen from nitrogen, oxygen and sulfur,
wherein R² , R³, R⁴ and R⁵ are independently selected from the group consisting of:
   - a hydrogen atom,
   - a halogen atom,
   - a hydroxyl group,
   - a C1-C20 alkyl group, linear or cyclic or branched, saturated or unsaturated, optionally substituted and/or optionally comprising a heteroatom,
   - an aryl group, optionally substituted by at least one C1-C20 alkyl group, linear or cyclic or branched, saturated or unsaturated, optionally substituted and/or optionally comprising at least one heteroatom,
   - an ester group of formula -C(O)O-Rₐ or of formula -C(O)O-Rₐ', Rₐ and Rₐ' being independently selected from the group consisting of a C1-C30 alkyl group, linear or cyclic or branched, saturated or unsaturated, optionally substituted and/or optionally comprising at least one heteroatom, or Rₐ being a C1-C30 alkylaryl group, wherein the alkyl is linear or cyclic or branched, saturated or unsaturated, linked to the aryl group, optionally substituted and/or optionally comprising at least one heteroatom;
   - a carboxyl group (-COOH),
   - a nitro group (-NO₂), and
   - a cyano group (-CN),
wherein R₄ and R₅ optionally form together a heterocycle,
and/or a salt thereof and/or a solvate thereof.

According to the invention, an "alkyl group" represents, unless expressly specified, a hydrocarbonated group, linear or branched, saturated or comprising at least one unsaturation.

According to the invention, a "hydrocarbonated group" is a group comprising or consisting in hydrogen and carbon atoms.

According to the invention, an "aryl group" represents an aromatic radical, unless expressly specified. An example of aryl group is a monocyclic aromatic radical comprising 6 carbon atoms.

According to the invention, a "heteroatom" represents, unless expressly specified, an atom selected from the group consisting of oxygen, nitrogen, sulfur and phosphorus.

According to the invention, a "heterocycle" represents, unless expressly specified, a cyclic moiety, saturated or unsaturated, comprising from 2 to 7 carbon atoms and at least one heteroatom chosen from nitrogen, oxygen and sulfur.

According to the invention, a "substituted" group or moiety means that at least one hydrogen radical of said group or moiety is replaced with an atom or a group of atoms called substituent.

Examples of preferred substituents are halogen (chloro, iodo, bromo, or fluoro); alkyl; alkenyl; alkynyl; hydroxy; alkoxy; nitro; thiol; thioether; imine; cyano; amido; phosphonato; phosphine; carboxyl; thiocarbonyl; sulfonyl; sulfonamide; ketone; aldehyde; ester; oxygen (-O); haloalkyl (e.g., trifluoromethyl); cycloalkyl, which may be monocyclic or fused or non-fused polycyclic (e.g., cyclopropyl, cyclobutyl, cyclopentyl, or cyclohexyl), or a heterocycloalkyl, which may be monocyclic or fused or non-fused polycyclic (e.g., pyrrolidinyl, piperidinyl, piperazinyl, morpholinyl, or thiazinyl), monocyclic or fused or non-fused polycyclic aryl or heteroaryl (e.g., phenyl, naphthyl, pyrrolyl, indolyl, furanyl, thiophenyl, imidazolyl, oxazolyl, isoxazolyl, thiazolyl, triazolyl, tetrazolyl, pyrazolyl, pyridyl, quinolinyl, isoquinolinyl, acridinyl, pyrazinyl, pyridazinyl, pyrimidinyl, benzimidazolyl, benzothiophenyl, or benzofuranyl); amino (primary, secondary, or tertiary); CO₂CH₃; CONH₂; OCH₂CONH₂; NH₂; SO₂NH₂; OCHF₂; CF₃; OCF₃; and such moieties may also be optionally substituted by a fused-ring structure or bridge, for example -OCH₂O-. These substituents may optionally be further substituted with a substituent selected from such groups.

According to the invention, an alkyl group "comprising at least one heteroatom" means that at least one hydrogen of said alkyl group is replaced by a substituent comprising at least on heteroatom, and/or that at least one carbon atom of said alkyl group is replaced by a heteroatom. Examples of alkyl groups comprising at least one heteroatom are alkyl groups comprising a primary, secondary or tertiary amine, an amido group, an azo group, an nitrile group, an imino group, an imido group, a azo group, a cyano group, a nitrile group, an aldehyde, a ketone, an ether, an ester group, an alcoxy group, a carbonate group, a carboxylic acid group, a peroxide group, a thiol group, a sulfide group, a disulfide group, a sulfoxide group, a sulfone group, a sulfonic acid group, a sulfinic acid group, a sulfonate ester group, a thiocyanato group, a thioketone, a thial, a thioester group, heterocycles internal to the alkyl group and heterocycles as substituents to the alkyl group.

According to an embodiment, a C1-C20 alkyl group, linear or cyclic or branched, saturated or unsaturated, optionally substituted and/or optionally comprising a heteroatom comprises at least an unsaturation and at least an ester group. An example of such C1-C20 alkyl group, linear or cyclic or branched, saturated or unsaturated, optionally substituted and/or optionally comprising a heteroatom is -CH=CH-C(O)O-tBu.

According to an embodiment, the compound of formula (I) according to the invention is a compound of formula (I') as follows: 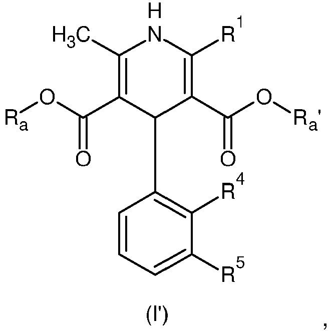 wherein R¹, Rₐ, Rₐ', R⁴ and R⁵ are as defined above.

According to an embodiment, the compound for use as an inhibitor of the initiation step of the reverse transcription of RNA of a type 1 human immunodeficiency virus (HIV-1) is a compound of formula (I) or (I'), wherein R¹ is selected form the group consisting of:
- an amino group NH₂,
- a C1-C10 alkyl group, linear or branched, saturated, and
- a C1-C10 alkyl group comprising at least one heteroatom chosen from oxygen and sulfur, and a terminal -NH₂ amino group.

According to an embodiment, the compound for use as an inhibitor of the initiation step of the reverse transcription of RNA of a type 1 human immunodeficiency virus (HIV-1) is a compound of formula (I) wherein R² and R³ are each independently an ester group of formula -C(O)O-Rₐ or of formula -C(O)O-Rₐ', or a compound of formula (I'),
wherein Rₐ and Rₐ' are independently selected from the group consisting of:
- a C1-C25 alkyl group Rₐ₁ optionally comprising at least one heteroatom chosen from nitrogen, oxygen and sulfur, said alkyl group Rₐ₁ being optionally substituted by at least one phenyl group, said phenyl group being optionally substituted by an halogen atom,
- a C1-C25 alkyl group Rₐ₂ comprising at least one heterocycle, said alkyl group Rₐ₂ being optionally substituted by at least one phenyl group, said phenyl group being optionally substituted by an halogen atom, and
- a heterocycle Rₐ₃ optionally substituted by at least one C1-C10 alkyl group optionally comprising at least one heteroatom chosen from nitrogen, oxygen and sulfur and/or optionally substituted by at least one terminal phenyl group.

According to an embodiment, the compound for use as an inhibitor of the initiation step of the reverse transcription of RNA of a type 1 human immunodeficiency virus (HIV-1) is a compound of formula (I) or (I'), wherein R₄ and R₅ are independently selected from the group consisting of:
- a hydrogen atom
- a halogen atom,
- a hydroxyl group,
- an C1-C20 alkyl group, linear or cyclic or branched, saturated or unsaturated, optionally comprising at least one heteroatom chosen from nitrogen, oxygen and sulfur and/or optionally substituted by a halogen atom,
- a carboxyl group (-COOH),
- a nitro group (-NO₂), and
- a cyano group (-CN),
or R₄ and R₅ form together a heterocycle.

According to an embodiment, the compound for use as an inhibitor of the initiation step of the reverse transcription of RNA of a type 1 human immunodeficiency virus (HIV-1) is a compound of formula (I) wherein R² and R³ are each independently an ester group of formula -C(O)O-Rₐ or of formula -C(O)O-Rₐ', or a compound of formula (I'),
wherein R¹ is selected form the group consisting of:
   - an amino group NH₂,
   - a C1-C10 alkyl group, linear or branched, saturated, and
   - a C1-C10 alkyl group comprising at least one heteroatom chosen from oxygen and sulfur, and a terminal -NH₂ amino group,
wherein Rₐ and Rₐ' are independently selected from the group consisting of:
   - a C1-C25 alkyl group Rₐ₁ optionally comprising at least one heteroatom chosen from nitrogen, oxygen and sulfur, said alkyl group Rₐ₁ being optionally substituted by at least one phenyl group, said phenyl group being optionally substituted by an halogen atom,
   - a C1-C25 alkyl group Rₐ₂ comprising at least one heterocycle, said alkyl group Rₐ₂ being optionally substituted by at least one phenyl group, said phenyl group being optionally substituted by an halogen atom, and
   - a heterocycle Rₐ₃ optionally substituted by at least one C1-C10 alkyl group optionally comprising at least one heteroatom chosen from nitrogen, oxygen and sulfur and/or optionally substituted by at least one terminal phenyl group, and
wherein R₄ and R₅ are independently selected from the group consisting of:
   - a hydrogen atom
   - a halogen atom,
   - a hydroxyl group,
   - an C1-C20 alkyl group, linear or cyclic or branched, saturated or unsaturated, optionally comprising at least one heteroatom chosen from nitrogen, oxygen and sulfur and/or optionally substituted by a halogen atom,
   - a carboxyl group (-COOH),
   - a nitro group (-NO₂), and
   - a cyano group (-CN),
or R₄ and R₅ form together a heterocycle.

According to an embodiment, the compound for use as an inhibitor of the initiation step of the reverse transcription of RNA of a type 1 human immunodeficiency virus (HIV-1) is a compound of formula (I) or (I'), wherein R¹ is selected form the group consisting of a methyl group CH₃ and an amino group NH₂, preferably R¹ is a methyl group.

According to an embodiment, the compound for use as an inhibitor of the initiation step of the reverse transcription of RNA of a type 1 human immunodeficiency virus (HIV-1) is a compound of formula (I) wherein R² and R³ are each independently an ester group of formula -C(O)O-Rₐ or of formula -C(O)O-Rₐ', or a compound of formula (I'),
wherein Rₐ and Rₐ' are independently selected from the group consisting of:
- a C1-C10 alkyl group optionally comprising at least one oxygen atom,
- a C1-C25 alkyl group substituted by one or two terminal phenyl groups and optionally comprising at least one nitrogen atom,
- a C1-C15 alkyl group comprising at least one saturated heterocycle comprising from 2 to 7 carbon atoms and at least one nitrogen atom, said saturated heterocycle being substituted by at least one phenyl group and/or by at least one C1-C10 alkyl group optionally substituted by one or two terminal phenyl groups, and
- a saturated heterocycle comprising from 2 to 7 carbon atoms and at least one nitrogen atom, said heterocycle being substituted by at least one phenyl group and/or by at least one C1-C10 alkyl group optionally substituted by one or two terminal phenyl groups.

According to an embodiment, the compound for use as an inhibitor of the initiation step of the reverse transcription of RNA of a type 1 human immunodeficiency virus (HIV-1) is a compound of formula (I) or (I'), wherein R₄ is hydrogen and R₅ is different from hydrogen, or R₅ is hydrogen and R₄ is different from hydrogen.

According to an embodiment, the compound for use as an inhibitor of the initiation step of the reverse transcription of RNA of a type 1 human immunodeficiency virus (HIV-1) is a compound of formula (I) wherein R² and R³ are each independently an ester group of formula -C(O)O-Rₐ or of formula -C(O)O-Rₐ', or a compound of formula (I'),
wherein R¹ is selected form the group consisting of:
   - an amino group NH₂,
   - a C1-C10 alkyl group, linear or branched, saturated, and
   - a C1-C10 alkyl group comprising at least one heteroatom chosen from oxygen and sulfur, and a terminal -NH₂ amino group,
wherein Rₐ and Rₐ' are independently selected from the group consisting of:
   - a C1-C10 alkyl group optionally comprising at least one oxygen atom,
   - a C1-C25 alkyl group substituted by one or two terminal phenyl groups and optionally comprising at least one nitrogen atom,
   - a C1-C15 alkyl group comprising at least one saturated heterocycle comprising from 2 to 7 carbon atoms and at least one nitrogen atom, said saturated heterocycle being substituted by at least one phenyl group and/or by at least one C1-C10 alkyl group optionally substituted by one or two terminal phenyl groups, and
   - a saturated heterocycle comprising from 2 to 7 carbon atoms and at least one nitrogen atom, said heterocycle being substituted by at least one phenyl group and/or by at least one C1-C10 alkyl group optionally substituted by one or two terminal phenyl groups, and
wherein R₄ is hydrogen and R₅ is different from hydrogen, or R₅ is hydrogen and R₄ is different from hydrogen.

According to an embodiment, the compound for use as an inhibitor of the initiation step of the reverse transcription of RNA of a type 1 human immunodeficiency virus (HIV-1) is a compound of formula (I) wherein R² and R³ are each independently an ester group of formula -C(O)O-Rₐ or of formula -C(O)O-Rₐ', or a compound of formula (I'),
wherein R¹ is selected form the group consisting of a methyl group CH₃ and an amino group NH₂, preferably R¹ is a methyl group,
wherein Rₐ and Rₐ' are independently selected from the group consisting of:
   - a C1-C10 alkyl group optionally comprising at least one oxygen atom,
   - a C1-C25 alkyl group substituted by one or two terminal phenyl groups and optionally comprising at least one nitrogen atom,
   - a C1-C15 alkyl group comprising at least one saturated heterocycle comprising from 2 to 7 carbon atoms and at least one nitrogen atom, said saturated heterocycle being substituted by at least one phenyl group and/or by at least one C1-C10 alkyl group optionally substituted by one or two terminal phenyl groups, and
   - a saturated heterocycle comprising from 2 to 7 carbon atoms and at least one nitrogen atom, said heterocycle being substituted by at least one phenyl group and/or by at least one C1-C10 alkyl group optionally substituted by one or two terminal phenyl groups, and
wherein R₄ is hydrogen and R₅ is different from hydrogen, or R₅ is hydrogen and R₄ is different from hydrogen.

According to an embodiment, the compound for use as an inhibitor of the initiation step of the reverse transcription of RNA of a type 1 human immunodeficiency virus (HIV-1) is a compound of formula (I) wherein R² and R³ are each independently an ester group of formula -C(O)O-Rₐ or of formula -C(O)O-Rₐ', or a compound of formula (I'),
wherein Rₐ and Rₐ' are independently selected from the group consisting of:
- a C1-C5 alkyl group, linear or branched, saturated, optionally comprising at least one oxygen atom,
- a C1-C10 alkyl group, linear, saturated or unsaturated, substituted by one or two terminal phenyl group(s),
- a C1-C5 alkyl group, linear or branched, saturated, substituted by one or two terminal phenyl group(s) and comprising at least one nitrogen atom,
- a C1-C10 alkyl group, comprising at least one saturated heterocycle comprising from 3 to 5 carbon atoms and one or two nitrogen atoms, said saturated heterocycle being substituted by -CHPh₂ or -CH₂Ph, and
- a saturated heterocycle comprising from 3 to 5 carbon atoms and one or two nitrogen atoms, said saturated heterocycle being substituted by -CHPh₂ or -CH₂Ph.

According to an embodiment, the compound for use as an inhibitor of the initiation step of the reverse transcription of RNA of a type 1 human immunodeficiency virus (HIV-1) is a compound of formula (I) or (I'), wherein R₄ and R₅ are independently selected from the group consisting of:
- a hydrogen atom,
- a halogen atom,
- a C1-C20 alkyl group, linear or branched, saturated or unsaturated, comprising at least one ester function, and
- a nitro group (-NO₂).

According to an embodiment, the compound for use as an inhibitor of the initiation step of the reverse transcription of RNA of a type 1 human immunodeficiency virus (HIV-1) is a compound of formula (I) wherein R² and R³ are each independently an ester group of formula -C(O)O-Rₐ or of formula -C(O)O-Rₐ', or a compound of formula (I'),
wherein R¹ is selected form the group consisting of:
   - an amino group NH₂,
   - a C1-C10 alkyl group, linear or branched, saturated, and
   - a C1-C10 alkyl group comprising at least one heteroatom chosen from oxygen and sulfur, and a terminal -NH₂ amino group,
wherein Rₐ and Rₐ' are independently selected from the group consisting of:
   - a C1-C5 alkyl group, linear or branched, saturated, optionally comprising at least one oxygen atom,
   - a C1-C10 alkyl group, linear, saturated or unsaturated, substituted by one or two terminal phenyl group(s),
   - a C1-C5 alkyl group, linear or branched, saturated, substituted by one or two terminal phenyl group(s) and comprising at least one nitrogen atom,
   - a C1-C10 alkyl group, comprising at least one saturated heterocycle comprising from 3 to 5 carbon atoms and one or two nitrogen atoms, said saturated heterocycle being substituted by -CHPh₂ or -CH₂Ph, and
   - a saturated heterocycle comprising from 3 to 5 carbon atoms and one or two nitrogen atoms, said saturated heterocycle being substituted by -CHPh₂ or -CH₂Ph, and
wherein R₄ and R₅ are independently selected from the group consisting of:
   - a hydrogen atom,
   - a halogen atom,
   - a C1-C20 alkyl group, linear or branched, saturated or unsaturated, comprising at least one ester function, and
   - a nitro group (-NO₂).

According to an embodiment, the compound for use as an inhibitor of the initiation step of the reverse transcription of RNA of a type 1 human immunodeficiency virus (HIV-1) is a compound of formula (I) wherein R² and R³ are each independently an ester group of formula -C(O)O-Rₐ or of formula -C(O)O-Rₐ', or a compound of formula (I'),
wherein R¹ is selected form the group consisting of a methyl group CH₃ and an amino group NH₂, preferably R¹ is a methyl group,
wherein Rₐ and Rₐ' are independently selected from the group consisting of:
- a C1-C5 alkyl group, linear or branched, saturated, optionally comprising at least one oxygen atom,
- a C1-C10 alkyl group, linear, saturated or unsaturated, substituted by one or two terminal phenyl group(s),
- a C1-C5 alkyl group, linear or branched, saturated, substituted by one or two terminal phenyl group(s) and comprising at least one nitrogen atom,
- a C1-C10 alkyl group, comprising at least one saturated heterocycle comprising from 3 to 5 carbon atoms and one or two nitrogen atoms, said saturated heterocycle being substituted by -CHPh₂ or -CH₂Ph, and
- a saturated heterocycle comprising from 3 to 5 carbon atoms and one or two nitrogen atoms, said saturated heterocycle being substituted by -CHPh₂ or -CH₂Ph, and
wherein R₄ and R₅ are independently selected from the group consisting of:
- a hydrogen atom,
- a halogen atom,
- a C1-C20 alkyl group, linear or branched, saturated or unsaturated, comprising at least one ester function, and
- a nitro group (-NO₂).

According to an embodiment, the compound for use as an inhibitor of the initiation step of the reverse transcription of RNA of a type 1 human immunodeficiency virus (HIV-1) is selected from the group consisting of: 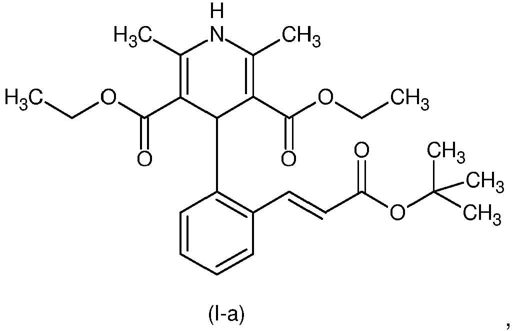 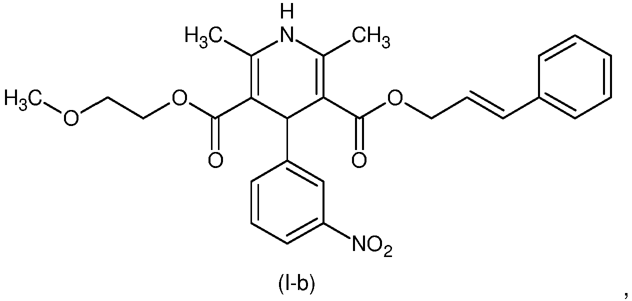 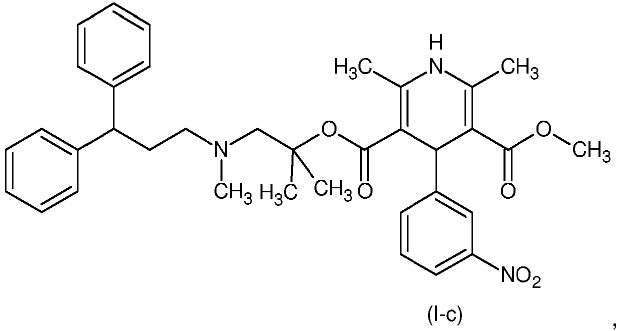 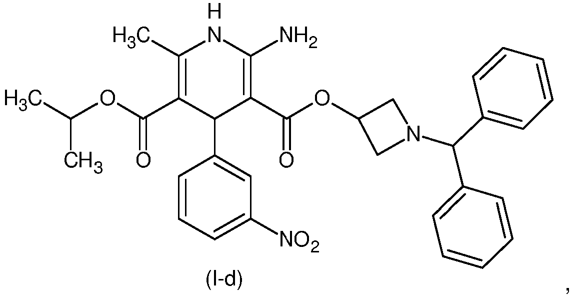 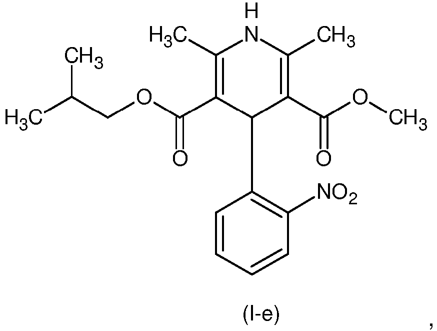 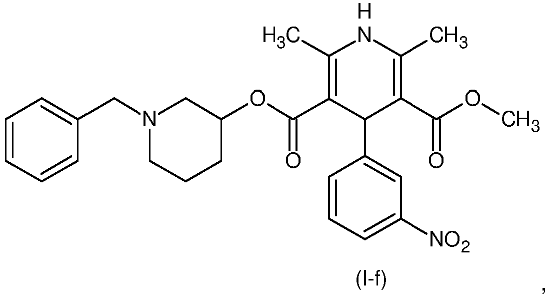 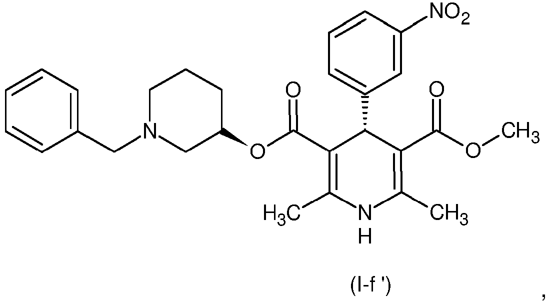 and

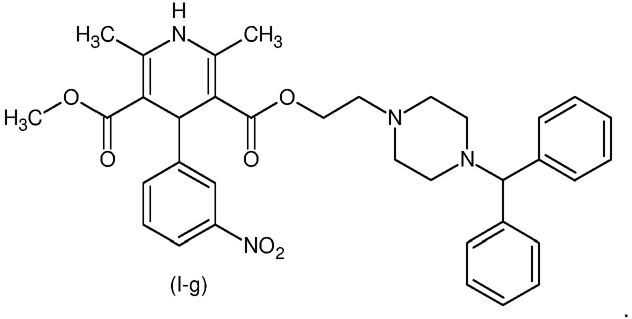

Preferably, the compound is a compound of formula (I) or (I') is selected from the group consisting of compound (I-a) and compound (I-b).

The present invention also concerns a compound of formula (II) for use as an inhibitor of the initiation step of the reverse transcription of RNA of a type 1 human immunodeficiency virus (HIV-1), preferably for use in a method of treatment of type 1 human immunodeficiency virus (HIV-1), wherein said formula (II) is as follows: 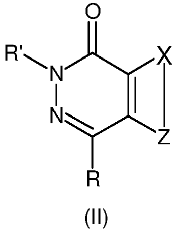
wherein X and Z together represent a fused benzene ring, optionally substituted by at least one substituent selected from the group consisting of:
   - a hydrogen atom,
   - a halogen atom,
   - a hydroxyl group,
   - a carboxyl group (-COOH),
   - a nitro group (-NO₂),
   - a cyano group (-CN),
   - a C1-C20 alkyl group, linear or cyclic or branched, saturated or unsaturated, optionally substituted and/or optionally comprising at least one heteroatom, and
   - an aryl group, optionally substituted,
wherein R and R' are independently selected form the group consisting of:
   - a hydrogen atom,
   - a halogen atom,
   - a hydroxyl group,
   - a carboxyl group (-COOH),
   - a nitro group (-NO₂),
   - a cyano group (-CN),
   - an amino group (-NH₂),
   - a C1-C20 alkyl group, linear or cyclic or branched, saturated or unsaturated, optionally comprising an heteroatom and/or optionally comprising at least one internal or terminal aryl group, said aryl group being optionally substituted by at least one substituent selected from the group consisting of a halogen atom, a hydroxyl group, a carboxyl group, a nitro group, a cyano group and a C1-C10 alkyl group,
   - a C1-C20 alkyl group comprising at least one internal or terminal heterocycle, said heterocycle being optionally substituted by at least one C1-C10 alkyl group,
   - an aryl group, optionally substituted by at least one substituent selected from the group consisting of a halogen atom, a hydroxyl group, a C1-C20 alkyl group optionally comprising a halogen atom and/or an heteroatom, a carboxyl group, a nitro group and a cyano group, and
   - a heterocycle optionally substituted by at least one C1-C10 alkyl group,
and/or a salt thereof and/or a solvate thereof.

According to an embodiment, the compound of formula (II) according to the invention is a compound of formula (II') as follows : 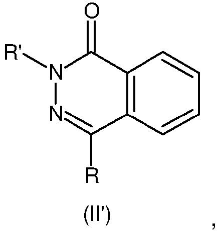 wherein R and R' are as defined above.

According to an embodiment, the compound for use as an inhibitor of the initiation step of the reverse transcription of RNA of a type 1 human immunodeficiency virus (HIV-1) is a compound of formula (II) or (II'), wherein R is selected form the group consisting of:
- a C1-C20 alkyl group, linear or cyclic or branched, saturated or unsaturated optionally comprising an heteroatom and/or optionally comprising at least one internal or terminal aryl group, said aryl group being optionally substituted by at least one substituent selected from the group consisting of a halogen atom, a hydroxyl group, a carboxyl group, a nitro group, a cyano group and a C1-C20 alkyl group, and
- an aryl group, optionally substituted by at least one substituent selected from the group consisting of a halogen atom, a hydroxyl group, a C1-C20 alkyl group optionally comprising a halogen atom and/or an heteroatom, a carboxyl group, a nitro group and a cyano group.

According to an embodiment, the compound for use as an inhibitor of the initiation step of the reverse transcription of RNA of a type 1 human immunodeficiency virus (HIV-1) is a compound of formula (II) or (II'), wherein R' is selected form the group consisting of:
- a C1-C20 alkyl group comprising at least one internal or terminal saturated heterocycle comprising from 2 to 7 carbon atoms and at least one nitrogen atom, said heterocycle being optionally substituted by at least one C1-C10 alkyl group, and
- a saturated heterocycle comprising from 2 to 7 carbon atoms and at least one nitrogen atom, being optionally substituted by at least one C1-C10 alkyl group.

According to an embodiment, the compound for use as an inhibitor of the initiation step of the reverse transcription of RNA of a type 1 human immunodeficiency virus (HIV-1) is a compound of formula (II) or (II'),
wherein R is selected form the group consisting of:
   - a C1-C20 alkyl group, linear or cyclic or branched, saturated or unsaturated optionally comprising an heteroatom and/or optionally comprising at least one internal or terminal aryl group, said aryl group being optionally substituted by at least one substituent selected from the group consisting of a halogen atom, a hydroxyl group, a carboxyl group, a nitro group, a cyano group and a C1-C20 alkyl group, and
   - an aryl group, optionally substituted by at least one substituent selected from the group consisting of a halogen atom, a hydroxyl group, a C1-C20 alkyl group optionally comprising a halogen atom and/or an heteroatom, a carboxyl group, a nitro group and a cyano group, and
wherein R' is selected form the group consisting of:
   - a C1-C20 alkyl group comprising at least one internal or terminal saturated heterocycle comprising from 2 to 7 carbon atoms and at least one nitrogen atom, said heterocycle being optionally substituted by at least one C1-C10 alkyl group, and
   - a saturated heterocycle comprising from 2 to 7 carbon atoms and at least one nitrogen atom, being optionally substituted by at least one C1-C10 alkyl group.

According to an embodiment, the compound for use as an inhibitor of the initiation step of the reverse transcription of RNA of a type 1 human immunodeficiency virus (HIV-1) is a compound of formula (II) or (II'), wherein R is a -CH₂-Ph group, Ph being an aryl group, optionally substituted by at least one substituent selected from the group consisting of a halogen atom, a hydroxyl group, a C1-C20 alkyl group, a carboxyl group, a nitro group and a cyano group.

According to an embodiment, the compound for use as an inhibitor of the initiation step of the reverse transcription of RNA of a type 1 human immunodeficiency virus (HIV-1) is a compound of formula (II) or (II'), wherein R' is a saturated heterocycle comprising from 2 to 7 carbon atoms and at least one nitrogen atom, said heterocycle being optionally substituted by at least one C1-C10 alkyl group.

According to an embodiment, the compound for use as an inhibitor of the initiation step of the reverse transcription of RNA of a type 1 human immunodeficiency virus (HIV-1) is a compound of formula (II) or (II'),
wherein R is a -CH₂-Ph group, Ph being an aryl group, optionally substituted by at least one substituent selected from the group consisting of a halogen atom, a hydroxyl group, a C1-C20 alkyl group, a carboxyl group, a nitro group and a cyano group, and
wherein R' is a saturated heterocycle comprising from 2 to 7 carbon atoms and at least one nitrogen atom, said heterocycle being optionally substituted by at least one C1-C10 alkyl group.

According to an embodiment, the compound for use as an inhibitor of the initiation step of the reverse transcription of RNA of a type 1 human immunodeficiency virus (HIV-1) has the following structure:

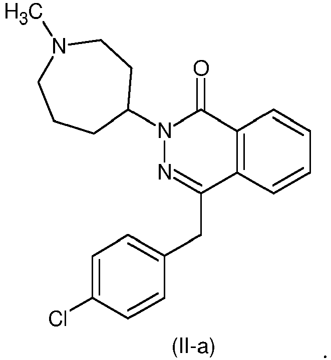

The invention includes all pharmaceutically acceptable salt forms of the compounds of formula (I) and (I') and of formula (II) and (II'). Pharmaceutically acceptable salts are those in which the counter ions do not contribute significantly to the physiological toxicity of the compounds and comprises pharmacological equivalents.

Some of the compounds of formula (I) and (I') and of formula (II) and (II') exist in stereoisomeric forms. The invention includes all stereoisomeric forms of the compounds of formula (I) and (I') and of formula (II) and (II') including enantiomers, diastereromers and tautomers.

The present invention also concerns the compound of formula (I), the compound of formula (I'), the compound of formula (II) and/or the compound of formula (II') for use in the treatment of the acquired immune deficiency syndrome.

The present invention also concerns a method of therapeutic treatment comprising the administration of an effective amount of an inhibitor according to the invention or of a compound of formula (I) or (I') and/or (II) or (II') to a subject in need thereof, in particular to a human or animal.

The invention also relates to a pharmaceutical composition or kit comprising a compound of formula (I) or (I') and/or (II) or (II') as defined according to the present invention for use as an inhibitor of the initiation step of the reverse transcription of RNA of a type 1 human immunodeficiency virus (HIV-1).

Preferably, the pharmaceutical composition or kit comprising comprising a compound of formula (I) or (I') and/or (II) or (II') as defined according to the present invention is for use in a method for treating a type 1 human immunodeficiency virus (HIV-1).

The invention will be better understood from reading the following non-limiting examples.

### FIGURES

Figure 1 is a graph representing the ex-vivo evolution of the HIV-1 replication after 5 days in contact with compounds (I-a), (I-b) and (II-a) at different concentrations, and in control conditions for comparative purpose.
Figure 2 is a graph representing the ex-vivo evolution of the HIV-1 cell survival after 5 days in contact with compounds (I-a), (I-b) and (II-a) at different concentrations, and in control conditions for comparative purpose.

### EXAMPLES

### Materials and methods

Compound of formula I-a was purchase from Prestwick (commercial reference: Prestw-1297) or from Sigma-Aldrich (commercial reference: SML0946).

Compound of formula I-b was purchase from Prestwick (commercial reference: Prestw-1376) or from Sigma-Aldrich (commercial reference: C1493).

Compound of formula II-a was purchase from Prestwick (commercial reference: Prestw-1130) or from Sigma-Aldrich (commercial reference: A7611).

### In-vitro tests

Experiments were performed on the interaction between the HIV-1 integrase (IN) and the catalytic domain of the human mitochondrial lysyl-tRNA synthetase (mLysRS), which constitutes the main contribution to the GagPol:mLysRS interaction.

### Expression of H6-mLysRS-HA in E. coli and purification

The cDNA encoding the mitochondrial species of human lysyl-tRNA synthetase (mLysRS, from amino acid residues 31 to 625 of the pre-protein from transcript NM_001130089.2) was amplified by PCR with oligonucleotides 5'-GGGGCTAGCCAACTTGCTCCTTTCACAGCGCCT (SEQ ID_NO 1) and 5'-CCCCTCGAGCTAGGCATAATCTGGCACATCATAAGGGTAGACAGAACTGCCAACTGT TGT (SEQ ID_NO 2) and inserted into the pET28b plasmid (Novagen) digested with *Nhel* and *Xhol.* The sequence of the recombinant plasmid was verified by DNA sequencing. The encoded protein, named H6-mLysRS-HA (SEQ ID_NO 3), contains a N-terminal His-tag (MGSSHHHHHH) (SEQ ID_NO 4), followed by a thrombin cleavage site (SSGLVPRGSHMAS) (SEQ ID_NO 5), and a C-terminal HA-tag (YPYDVPDYA) (SEQ ID_NO 6).

The protein was expressed in *E. coli* BL21(DE3) grown in LB medium supplemented with kanamycin (50 µg/ml). Culture (3 liters) was grown at 37 °C to an A₆₀₀=0.25, transferred at 28 °C and grown to an A₆₀₀=0.5, and expression was induced by addition of 1 mM IPTG for 4 hours. Cells were collected by centrifugation (3,000 *g,* 10 min, 4 °C), washed twice with ice-cold buffer A (20 mM K-phosphate pH 7.5, 150 mM NaCl, 5% glycerol, 5 mM 2-mercaptoethanol) containing 10 mM imidazole, resuspended in the same buffer (1 ml per g of cell pellet) and lysed in an Eaton Press after freezing in dry ice. All subsequent steps were conducted at 4 °C. After addition of 2 vol. of buffer B (20 mM K-phosphate pH 7.5, 500 mM NaCl, 5% glycerol, 5 mM 2-mercaptoethanol) containing 10 mM imidazole, and of protease inhibitors (1 mM Pefabloc, 10 mM benzamidine and 10 mM PMSF), extracts were clarified by sonication and by centrifugation at 20,000 g for 20 min, and at 70,000 g for 1 hour.

The clear supernatant was applied to a 5 ml column of Ni-NTA Superflow (Qiagen), at 4 °C. The matrix was extensively washed with buffer B containing 10 mM imidazole, and elution was performed by a linear gradient of imidazole (10 to 500 mM) in buffer B. Fractions containing H6-mLysRS-HA were dialyzed against buffer AS (20 mM Tris-HCl pH 7.5, 50 mM KCI, 10% glycerol, 10 mM 2-mercaptoethanol), and were applied to a 1 ml column of Source 15S (GE Healthcare) equilibrated in the same buffer. Proteins were eluted by a linear gradient (50 column vol.) of KCI from 50 to 335 mM in the same buffer. Fractions containing H6-mLysRS-HA were dialyzed against storage buffer (25 mM K-phosphate pH 7.5, 55% glycerol, 2 mM DTT), and stored at -20 °C.

Protein concentration was determined by using a calculated absorption coefficient of 0.674 A₂₈₀ units·mg⁻¹·cm².

### Cleavage of H6-mLysRS-HA to give mLysRS-HA

A thrombin cleavage site is inserted between the N-terminal His-tag and the coding sequence of mLysRS-HA. The H6-tag was removed from the purified H6-mLysRS-HA protein to generate mLysRS-HA (SEQ ID_NO 7).

H6-mLysRS-HA was incubated at a concentration of 1 mg/ml, in the presence of thrombin (Roche) at a ratio 50:1 (w:w), in a buffer containing 50 mM Tris-HCl pH 8.0, 150 mM NaCl, 2.5 mM CaCl₂, 2 mM DTT, 10% glycerol. After 30 min at 25 °C, digestion was stopped by addition of Pefabloc at 2 mM. The loss of the H6-tag was monitored by Western blotting with anti-His antibodies (Qiagen #34660), and using the HTRF assay (see below). In this assay, when mLysRS-HA was incubated in the presence of anti His-tag and anti HA-tag antibodies, no HTRF signal was detected. The protein mLysRS-HA was stored at -20 °C after addition of 1 vol. of glycerol.

### Expression of HIV-1 integrase in insect cells and purification

Expression and purification of integrase from HIV-1 was conducted essentially as previously described (Khoder-Agha et al. BMC Biochemistry 2018, 19:2).

The HIV-1 integrase coding region from pNL4-3 (nucleotides 4230 to 5093) was amplified by PCR with oligonucleotides 5'-CGCGGATCCCGGTCCGAAGCGCGCGGAATTCATAATGGCGTTTTTAGATGGAATAG ATAAGG (SEQ ID_NO 8) and 5'-TCTCGACAAGCTTGGTACCGCATGCCTCGAGTTAGTGGTGGTGGTGGTGGTGATCC TCATCCTGTCTACTTG (SEQ ID_NO 9), and introduced in pFastBac1 (Life Technologies) digested with EcoRI and *Xhol.* The sequence of the recombinant plasmid was verified by DNA sequencing. A His-tag coding sequence has been appended at the C-terminus of integrase (the sequence of the resulting protein, named IN-H6, is SEQ ID_NO 10).

Recombinant bacmids and baculoviruses were obtained as previously described (Kobbi et al. Biochim Open. 2016, 2:52-61).

Baculoviruses were used to infect 8 liters of High Five cells (Life Technologies) grown in suspension in Express Five SFM medium (Life Technologies). After 68 h of culture at 27°C with constant orbital shaking at 110 rpm, cells were harvested by centrifugation, washed with ice-cold buffer A containing 10 mM imidazole, and the cell pellet was stored at -80 °C. Pellet was rapidly thawed at 37 °C and cells were lysed after addition of 120 ml of buffer A containing 10 mM imidazole and 1% Triton X-100, in the presence of 1 mM Pefabloc, 10 mM benzamidine and 10 mM PMSF. After addition of 240 ml of buffer B containing 50 mM imidazole, extract was clarified by centrifugation at *70,000* g for 30 min at 4 °C and incubated 1 h at 4 °C with 1 ml of Ni-NTA Superflow matrix (Qiagen). Beads were extensively washed with buffer B containing 50 mM imidazole, and elution was performed by adding 6 x 1 ml of buffer B containing 400 mM imidazole. Eluted proteins were dialyzed against buffer ASU (20 mM Tris-HCl pH 7.0, 1 M urea, 10 % glycerol, 1 mM EDTA, 10 mM 2-mercaptoethanol) containing 150 mM NaCl, and applied to a Mono S HR 5/5 column (GE Healthcare) equilibrated in the same buffer. Proteins were eluted by a linear gradient (40 column vol.) of NaCl from 150 to 450 mM in buffer ASU. Fractions containing integrase were concentrated by ultrafiltration (Vivaspin 6, 10 kDa), dialyzed against storage buffer (20 mM K-phosphate pH 7.5, 1 M NaCl, 2 mM DTT), and stored at -80 °C. Protein concentration was determined by using a calculated absorption coefficient of 1.529 A₂₈₀ units mg⁻¹ cm².

### Homogeneous time-resolved fluorescence assay

Homogeneous time-resolved fluorescence (HTRF) assays were performed in black, half-area, flat bottom, 96-well microplates (Costar #3694). Human mitochondrial LysRS with a C-terminal HA-tag (mLysRS-HA), diluted in HTRF buffer (10 mM Tris-HCl pH 7.5, 50 mM NaCl, 10 mM 2-mercaptoethanol, BSA at 1 mg/ml) was added (20 µl at a dimer concentration of 3.75 nM) in the wells of microplates placed on ice. Compounds of formula I or II (1 µl of a 10 mM solution in DMSO) were then added to the wells, and mixing was achieved by pipetting up and down 3-times 10 µl. After centrifugation at 900 g for 1 min at 4 °C, plates were placed at 4 °C on thermal modules of an *epMotion* 5075v automated pipetting system (Eppendorf). A 10 µl sample of IN-H6 diluted in HTRF buffer at a dimer concentration of 250 nM was added and mixing was achieved by pipetting up and down 3-times 15 µl. Incubation was conducted at 4 °C for 1 h.

A 10 µl aliquot of a mix of antibodies prepared in HTRF buffer, directed to the His-tag, conjugated with Eu³⁺ cryptate (Cisbio #61HISKLB, 0.125 µl per test, prepared as recommended by the supplier), and to the HA-tag, conjugated with XL665 (Cisbio #610HAXLB, 0.375 µl per test, prepared as recommended by the supplier), was added and mixing was achieved by pipetting up and down 3-times 15 µl. Incubation was conducted at 4 °C for 30 min.

A 10 µl solution of KF prepared in HTRF buffer at a concentration of 250 mM was then added and mixing was achieved by pipetting up and down 3-times 20 µl. After centrifugation at 900 g for 3 min at 4 °C, fluorescence of Eu³⁺ cryptate and of XL665 was recorded at 620 nm (*I*₆₂₀) and 665 nm (*I*₆₆₅), respectively, after excitation of Eu³⁺ cryptate at 317 nm, in an Infinite M1000 PRO microplate reader (TECAN). Results are expressed as the ratio of *I*₆₆₅/*I*₆₂₀. The HTRF signal corresponds to the ratio of fluorescence at 665 and 620 nm (I665/I620). HTRF (%) corresponds to normalization of the values (for each curve 100% corresponds to the value obtained without inhibitor).

### Control tests

Two control tests were performed to avoid false positives.

The first control test utilizes p38-H6, instead of IN-H6. This protein p38-H6 is the scaffold protein from the cytoplasmic human multisynthetase complex that interacts with the catalytic domain of LysRS at a site distinct from that involved in the interaction with IN-H6 (Kobbi et al. J. Mol. Biol. 2011, 410:875-886). The assay was performed as described above, except that a 10 µl sample of p38-H6 diluted in HTRF buffer at a dimer concentration of 50 nM was used, and anti HA-tag antibodies, conjugated with Eu³⁺ cryptate (Cisbio #610HAKLB, 0.125 µl per test, prepared as recommended by the supplier), and anti His-tag antibodies, conjugated with XL665 (Cisbio #61HISXLB, 0.125 µl per test, prepared as recommended by the supplier), were used.

In the second control, only H6-mLysRS-HA (a 30 µl sample diluted in HTRF buffer at a dimer concentration of 1.34 nM) was added in the assay, in the absence of a partner protein. Fluorescence energy transfer results from the binding of one antibody to the N-terminal His-tag, and of the second antibody to the C-terminal HA-tag. Thus, a compound reducing the HTRF signal by interfering with the fluorescence donor (Eu³⁺) or acceptor (XL665) should be eliminated.

### In-vitro determination of the half maximal inhibitory concentration (IC50)

IC50 of the compounds of formula I and II were determined in the HTRF assay after incubation of the protein partners in the presence of increasing concentrations of the compound of formula I or II (from 17 to 666 µM). IC50s were obtained by nonlinear regression of the theoretical equation to the experimental curve using the KaleidaGraph 4.5 software (Synergy Software).

### Ex-vivo inhibition of the HIV-1 virus replication

Ability of compounds of formula I or II to inhibit the HIV-1 replicative cycle ex vivo was conducted by methods commonly used in antiretroviral pharmacology.

### Cells and Viruses.

MT4 cell line (Charneau et al. J. Virol. 1992, 66:2814) were maintained in RPMI 1640. HEK293T were maintained in Dulbecco's Modified Eagle Medium (DMEM). All media were supplemented with Glutamax and with 10% heat-inactivated fetal calf serum (Hyclone) and 1% penicillin/streptomycin (100 units/mL) (Gibco). All media were purchased from Gibco (Life Technologies Co.). All cell lines used here were incubated at 37 °C, under 5% CO₂ atmosphere. HIV-1 stocks were prepared by calcium phosphate-mediated transfection of HEK293T cells, as previously described (Manic et al. Human gene therapy methods 2012, 23:84), with shuttle vector plasmids encoding HIV-1 NL4-3 (GenBank: AF324493.1) or NLENG1-IRES-GFP (Levy et al. Proc. Natl. Acad. Sci. USA 2004, 101:4204). The latter vector comes from HIV NL4-3 strain, and contains a *gfp-*IRES-*nef* cassette at the *nef* locus. For clarity reason, we designate this vector here as "HIV-1 *gfp*^{+"}*.*

The HIV-1 p24^{gag} antigen contents in viral inocula were determined by enzyme-linked immunosorbent assay (ELISA, Perkin-Elmer Life Sciences).

### HIV infectivity and toxicity assays.

Replication of the NL4-3 virus was determined by the ELISA technique. The cytotoxicity is evaluated by the MTT technique.

For HIV-1 *gfp+* vectors, viral replication is followed by GFP expression [i.e. the percentage and geometric mean fluorescence intensity (MFI) of GFP⁺ cells]. Infectivity was estimated by flow cytometry using a FACSCaliburTM cytofluorometer (BD Biosciences). Toxicity is also assessed by flow cytometry (side and forward scatter).

### Effect of drugs on multi-cycle virus replication.

At J0 (day zero) MT4 cells was used for infection with HIV *gfp+* virus at multiplicity of infection (m.o.i.) reaching 0.1-0.2 (50 ng of p24^{gag} antigen per 10⁶ cells). Two days post-infection (J2), MT4 cells are washed extensively (3 times with PBS) and resuspended using fresh RPMI medium (2 x 10⁵ cells per ml) and plated into 6 well plates (2 ml per well) in the presence of compounds of formula I or II at various molecules concentrations. A control not infected with the virus (MT4 Ninf), or brought into contact with DMSO (inf DMSO, the solvent of compounds of formula I or II), or with dolutegravir (DTG) at 100 nM, a known inhibitor of the integrase, are conducted in parallel. At time J5 (day five), cells are then collected for cytometry analysis to quantify the production of viral particles and cell survival.

### Example 1: Determination of the half maximal inhibitory concentration (IC50) of compounds of formula I or II regarding the mLysRS:IN interaction

IC50 of compounds of formula I-a, I-b, I-c, I-d, I-e, I-f, I-g, and II-a were determined by HTRF, as described above. As the control test, the mLysRS:p38 interaction was also determined.

The IC50 of compounds of formula I-a, I-b, I-c, I-d, I-e, I-f, I-g, and II-a are gathered in the table below.

| **Compound** | **IC50 (µM)** |
|---|---|
| I-a | 65±10 |
| I-b | 400±150 |
| I-c | 320±150 |
| I-d | 400±150 |
| I-e | 450±150 |
| I-f | 450±100 |
| I-g | 400±100 |
| II-a | 300±50 |

These results demonstrate that compounds of formula I and II inhibit the interaction between the HIV-1 integrase (IN) and the catalytic domain of the human mitochondrial lysyl-tRNA synthetase (mLysRS).

It was also demonstrated that compounds of formula I and II inhibit the mLysRS:Pol interaction with IC50 values comparable with those obtained for the mLysRS:IN interaction. Consequently, the inhibition of the mLysRS:IN interaction is sufficient to prevent the mLysRS:Pol association.

### Example 2: In-cellulo tests

Compounds of formula I-a, I-b and II-a were tested for their ability to inhibit the HIV-1 replicative cycle *ex vivo* following the method described above.

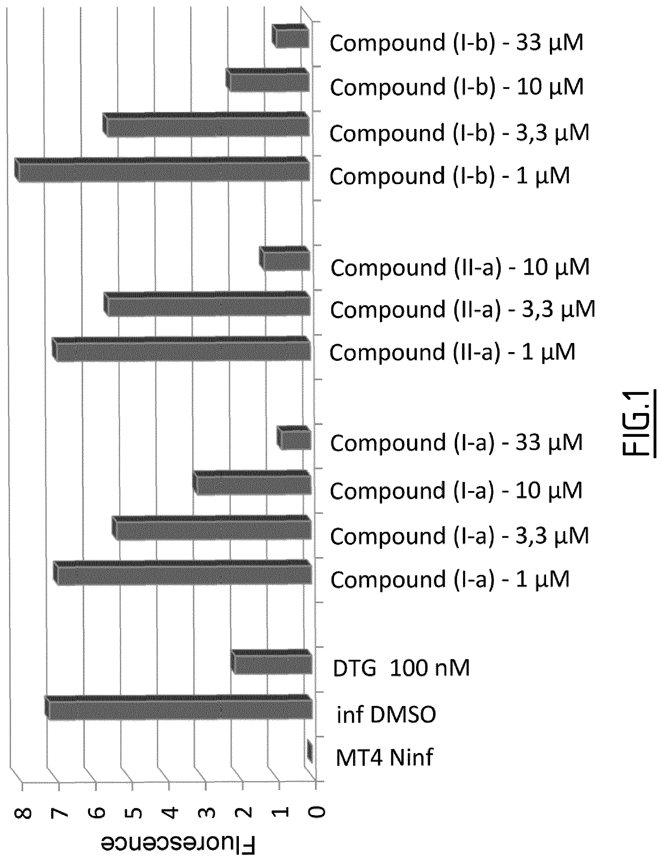

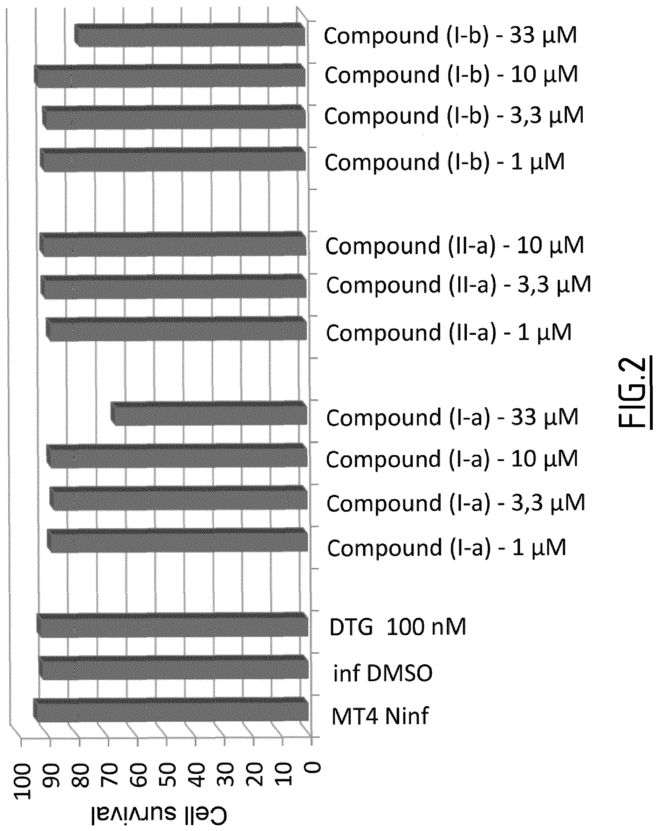

The results regarding the HIV-1 replication at day 5 are presented in figures 1 and 2.

At a concentration of 10 µM for compound II-a and of 33 µM for compounds I-a and I-b, the production of viral particles is greatly reduced. At the same time, no cellular toxicity has been observed up to 10 µM of compound of formula I-a, I-b or II-a.

These results demonstrate the anti-viral activity of the compounds of formula I and II.

## Claims

1. A compound of formula (I) for use in a method of treatment of type 1 human immunodeficiency virus (HIV-1), wherein said formula (I) is as follows: 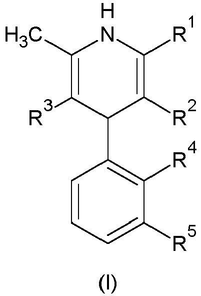
wherein R¹ is selected form the group consisting of:
- a hydrogen atom,
- an amino group NH₂, and
- a C1-C10 alkyl group, linear or cyclic or branched, saturated or unsaturated, optionally comprising an heteroatom chosen from nitrogen, oxygen and sulfur,
wherein R² , R³, R⁴ and R⁵ are independently selected from the group consisting of:
- a hydrogen atom,
- a halogen atom,
- a hydroxyl group,
- a C1-C20 alkyl group, linear or cyclic or branched, saturated or unsaturated, optionally substituted and/or optionally comprising a heteroatom,
- an aryl group, optionally substituted by at least one C1-C20 alkyl group, linear or cyclic or branched, saturated or unsaturated, optionally substituted and/or optionally comprising at least one heteroatom,
- an ester group of formula -C(O)O-Rₐ or of formula -C(O)O-Rₐ', Rₐ and Rₐ' being independently selected from the group consisting of a C1-C30 alkyl group, linear or cyclic or branched, saturated or unsaturated, optionally substituted and/or optionally comprising at least one heteroatom, or Rₐ being a C1-C30 alkylaryl group, wherein the alkyl is linear or cyclic or branched, saturated or unsaturated, linked to the aryl group, optionally substituted and/or optionally comprising at least one heteroatom;
- a carboxyl group (-COOH),
- a nitro group (-NO₂), and
- a cyano group (-CN),
wherein R₄ and R₅ optionally form together a heterocycle,
and/or a salt thereof and/or a solvate thereof.

2. The compound for use according to claim 1, wherein said compound is a compound of formula (I') as follows: 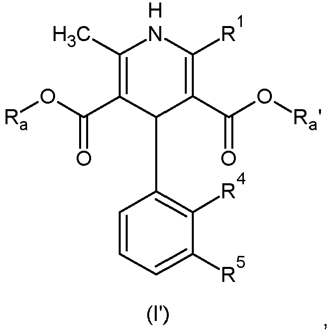 wherein R¹, Rₐ, Rₐ', R⁴ and R⁵ are as defined in claim 1.

3. The compound for use according to claim 1 or 2, wherein said compound is a compound of formula (I) wherein R² and R³ are each independently an ester group of formula -C(O)O-Rₐ or of formula-C(O)O-Rₐ', or a compound of formula (I'),
wherein Rₐ and Rₐ' are independently selected from the group consisting of:
- a C1-C25 alkyl group Rₐ₁ optionally comprising at least one heteroatom chosen from nitrogen, oxygen and sulfur, said alkyl group Rₐ₁ being optionally substituted by at least one phenyl group, said phenyl group being optionally substituted by an halogen atom,
- a C1-C25 alkyl group Rₐ₂ comprising at least one heterocycle, said alkyl group Rₐ₂ being optionally substituted by at least one phenyl group, said phenyl group being optionally substituted by an halogen atom, and
- a heterocycle Rₐ₃ optionally substituted by at least one C1-C10 alkyl group optionally comprising at least one heteroatom chosen from nitrogen, oxygen and sulfur and/or optionally substituted by at least one terminal phenyl group.

4. The compound according to any one of claims 1 to 3, wherein said compound is a compound of formula (I) or (I'), wherein R₄ and R₅ are independently selected from the group consisting of:
- a hydrogen atom
- a halogen atom,
- a hydroxyl group,
- an C1-C20 alkyl group, linear or cyclic or branched, saturated or unsaturated, optionally comprising at least one heteroatom chosen from nitrogen, oxygen and sulfur and/or optionally substituted by a halogen atom,
- a carboxyl group (-COOH),
- a nitro group (-NO₂), and
- a cyano group (-CN),
or R₄ and R₅ form together a heterocycle.

5. The compound for use according to any one of claims 1 to 4, wherein said compound is a compound of formula (I) or (I'), wherein R¹ is selected form the group consisting of a methyl group CH₃ and an amino group NH₂, preferably R¹ is a methyl group.

6. The compound for use according to any one of claims 1 to 5, wherein said compound is a compound of formula (I) wherein R² and R³ are each independently an ester group of formula -C(O)O-Rₐ or of formula -C(O)O-Rₐ', or a compound of formula (I'),
wherein Rₐ and Rₐ' are independently selected from the group consisting of:
- a C1-C10 alkyl group optionally comprising at least one oxygen atom,
- a C1-C25 alkyl group substituted by one or two terminal phenyl groups and optionally comprising at least one nitrogen atom,
- a C1-C15 alkyl group comprising at least one saturated heterocycle comprising from 2 to 7 carbon atoms and at least one nitrogen atom, said saturated heterocycle being substituted by at least one phenyl group and/or by at least one C1-C10 alkyl group optionally substituted by one or two terminal phenyl groups, and
- a saturated heterocycle comprising from 2 to 7 carbon atoms and at least one nitrogen atom, said heterocycle being substituted by at least one phenyl group and/or by at least one C1-C10 alkyl group optionally substituted by one or two terminal phenyl groups.

7. The compound for use according to any one of claims 1 to 6, wherein said compound is a compound of formula (I) or (I'), wherein R₄ is hydrogen and R₅ is different from hydrogen, or R₅ is hydrogen and R₄ is different from hydrogen.

8. The compound for use according to any one of claims 1 to 7, wherein said compound is a compound of formula (I) wherein R² and R³ are each independently an ester group of formula -C(O)O-Rₐ or of formula -C(O)O-Rₐ', or a compound of formula (I'),
wherein Rₐ and Rₐ' are independently selected from the group consisting of:
- a C1-C5 alkyl group, linear or branched, saturated, optionally comprising at least one oxygen atom,
- a C1-C10 alkyl group, linear, saturated or unsaturated, substituted by one or two terminal phenyl group(s),
- a C1-C5 alkyl group, linear or branched, saturated, substituted by one or two terminal phenyl group(s) and comprising at least one nitrogen atom,
- a C1-C10 alkyl group, comprising at least one saturated heterocycle comprising from 3 to 5 carbon atoms and one or two nitrogen atoms, said saturated heterocycle being substituted by -CHPh₂ or -CH₂Ph, and
- a saturated heterocycle comprising from 3 to 5 carbon atoms and one or two nitrogen atoms, said saturated heterocycle being substituted by -CHPh₂ or - CH₂Ph.

9. The compound for use according to any one of claims 1 to 8, wherein said compound is a compound of formula (I) or (I'), wherein R₄ and R₅ are independently selected from the group consisting of:
- a hydrogen atom,
- a halogen atom,
- a C1-C20 alkyl group, linear or branched, saturated or unsaturated, comprising at least one ester function, and
- a nitro group (-NO₂).

10. The compound for use according to any one of claims 1 to 9, wherein said compound is selected from the group consisting of: 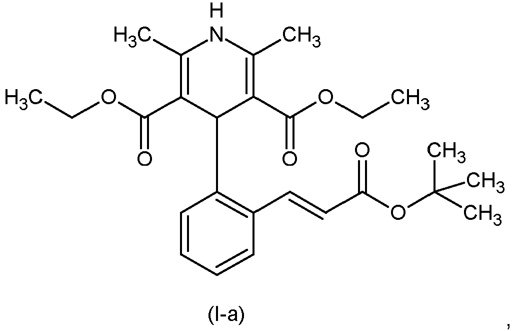 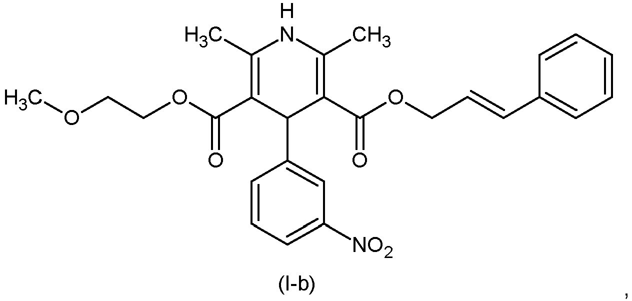 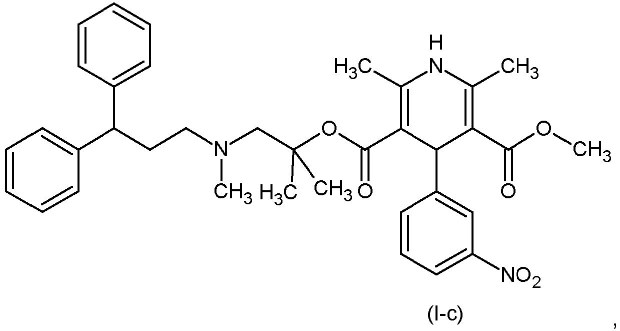 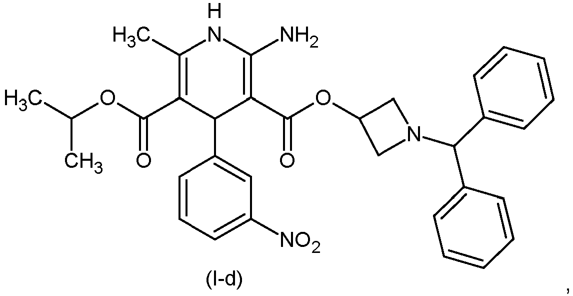 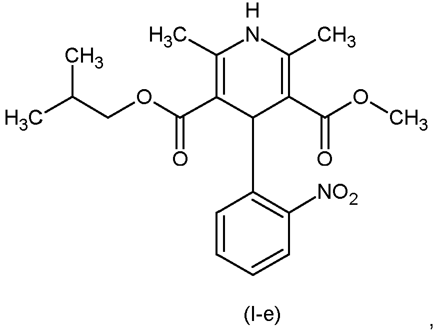 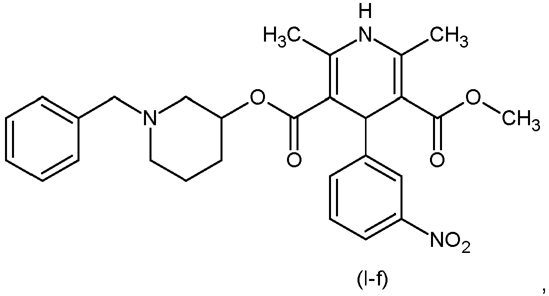 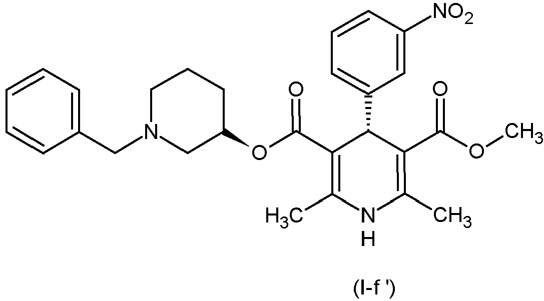 and

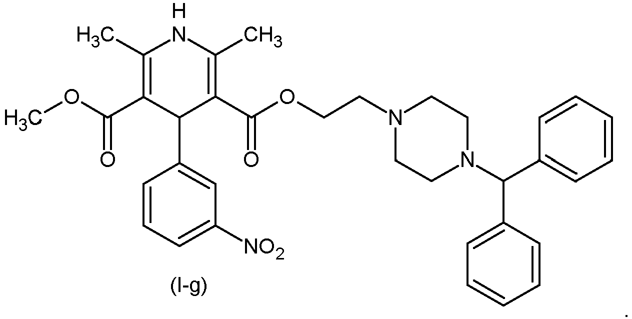

11. A compound of formula (II) for use in a method of treatment of type 1 human immunodeficiency virus (HIV-1), wherein said formula (II) is as follows: 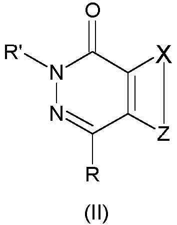
wherein X and Z together represent a fused benzene ring, optionally substituted by at least one substituent selected from the group consisting of:
- a hydrogen atom,
- a halogen atom,
- a hydroxyl group,
- a carboxyl group (-COOH),
- a nitro group (-NO₂),
- a cyano group (-CN),
- a C1-C20 alkyl group, linear or cyclic or branched, saturated or unsaturated, optionally substituted and/or optionally comprising at least one heteroatom, and
- an aryl group, optionally substituted,
wherein R and R' are independently selected form the group consisting of:
- a hydrogen atom,
- a halogen atom,
- a hydroxyl group,
- a carboxyl group (-COOH),
- a nitro group (-NO₂),
- a cyano group (-CN),
- an amino group (-NH₂),
- a C1-C20 alkyl group, linear or cyclic or branched, saturated or unsaturated, optionally comprising an heteroatom and/or optionally comprising at least one internal or terminal aryl group, said aryl group being optionally substituted by at least one substituent selected from the group consisting of a halogen atom, a hydroxyl group, a carboxyl group, a nitro group, a cyano group and a C1-C10 alkyl group,
- a C1-C20 alkyl group comprising at least one internal or terminal heterocycle, said heterocycle being optionally substituted by at least one C1-C10 alkyl group,
- an aryl group, optionally substituted by at least one substituent selected from the group consisting of a halogen atom, a hydroxyl group, a C1-C20 alkyl group optionally comprising a halogen atom and/or an heteroatom, a carboxyl group, a nitro group and a cyano group, and
- a heterocycle optionally substituted by at least one C1-C10 alkyl group, and/or a salt thereof and/or a solvate thereof.

12. The compound for use according to claim 11, wherein said compound is a compound of formula (II') as follows: 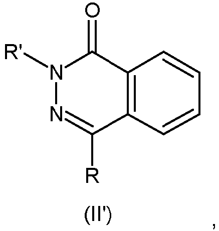 wherein R and R' are as defined in claim 11.

13. The compound for use according to claim 11 or 12, wherein said compound is a compound of formula (II) or (II'), wherein R is selected form the group consisting of:
- a C1-C20 alkyl group, linear or cyclic or branched, saturated or unsaturated optionally comprising an heteroatom and/or optionally comprising at least one internal or terminal aryl group, said aryl group being optionally substituted by at least one substituent selected from the group consisting of a halogen atom, a hydroxyl group, a carboxyl group, a nitro group, a cyano group and a C1-C20 alkyl group, and
- an aryl group, optionally substituted by at least one substituent selected from the group consisting of a halogen atom, a hydroxyl group, a C1-C20 alkyl group optionally comprising a halogen atom and/or an heteroatom, a carboxyl group, a nitro group and a cyano group.

14. The compound for use according to any one of claims 11, 12 or 13, wherein said compound is a compound of formula (II) or (II'), wherein R' is selected form the group consisting of:
- a C1-C20 alkyl group comprising at least one internal or terminal saturated heterocycle comprising from 2 to 7 carbon atoms and at least one nitrogen atom, said heterocycle being optionally substituted by at least one C1-C10 alkyl group, and
- a saturated heterocycle comprising from 2 to 7 carbon atoms and at least one nitrogen atom, being optionally substituted by at least one C1-C10 alkyl group.

15. The compound for use according to any one of claims 11 to 14, wherein said compound is a compound of formula (II) or (II'), wherein R is a -CH₂-Ph group, Ph being an aryl group, optionally substituted by at least one substituent selected from the group consisting of a halogen atom, a hydroxyl group, a C1-C20 alkyl group, a carboxyl group, a nitro group and a cyano group.

16. The compound for use according to any one of claims 11 to 15, wherein said compound is a compound of formula (II) or (II'), wherein R' is a saturated heterocycle comprising from 2 to 7 carbon atoms and at least one nitrogen atom, said heterocycle being optionally substituted by at least one C1-C10 alkyl group.

17. The compound for use according to any one of claims 11 to 16, wherein said compound has the following structure:

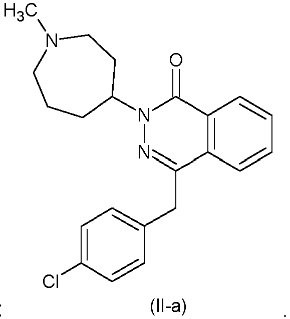

18. The compound for use according to any one of claims 1 to 17; wherein the treatment of type 1 human immunodeficiency virus is the treatment of the acquired immune deficiency syndrome.

19. A pharmaceutical composition or kit comprising a compound of formula (I) or (I') and/or (II) or (II') according to any one of claims 1 to 17 for use in a method for treating a type 1 human immunodeficiency virus (HIV-1).

## Patentansprüche

1. Verbindung der Formel (I) zur Verwendung in einem Verfahren zur Behandlung des humanen Immundefizienzvirus Typ 1 (HIV-1), wobei die Formel (I) wie folgt lautet: 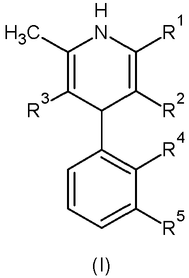
worin R¹ ausgewählt ist aus der Gruppe bestehend aus:
- ein Wasserstoffatom,
- eine Aminogruppe NH₂, und
- eine C1-C10-Alkylgruppe, linear oder zyklisch oder verzweigt, gesättigt oder ungesättigt, die gegebenenfalls ein Heteroatom umfasst, ausgewählt aus Stickstoff, Sauerstoff und Schwefel,
worin R², R³, R⁴ und R⁵ unabhängig voneinander ausgewählt sind aus der Gruppe bestehend aus:
- ein Wasserstoffatom,
- ein Halogenatom,
- eine Hydroxylgruppe,
- eine lineare oder cyclische oder verzweigte, gesättigte oder ungesättigte C1-C20-Alkylgruppe, die gegebenenfalls substituiert ist und/oder ein Heteroatom enthaltend,
- eine Arylgruppe, die gegebenenfalls durch mindestens eine lineare oder cyclische oder verzweigte, gesättigte oder ungesättigte C1-C20-Alkylgruppe substituiert ist, die gegebenenfalls substituiert ist und/oder mindestens ein Heteroatom enthaltend,
- eine Estergruppe der Formel -C(O)O-Ra oder der Formel -C(O)O-Ra', wobei Ra und Ra' unabhängig voneinander ausgewählt sind aus der Gruppe bestehend aus einer C1-C30-Alkylgruppe, linear oder zyklisch oder verzweigt, gesättigt oder ungesättigt, die gegebenenfalls substituiert ist und/oder mindestens ein Heteroatom enthaltend, oder Ra eine C1-C30-Alkylarylgruppe ist, wobei das Alkyl linear oder cyclisch oder verzweigt, gesättigt oder ungesättigt, an die Arylgruppe gebunden, gegebenenfalls substituiert ist und/oder mindestens ein Heteroatom enthaltend;
- eine Carboxylgruppe (-COOH),
- eine Nitrogruppe (-NO₂), und
- eine Cyanogruppe (-CN),
wobei R4 und R5 können zusammen einen Heterocyclus bilden, und/oder ein Salz davon und/oder ein Solvat davon.

2. Verbindung zur Verwendung nach Anspruch 1, wobei die Verbindung eine Verbindung der Formel (I') wie folgt ist 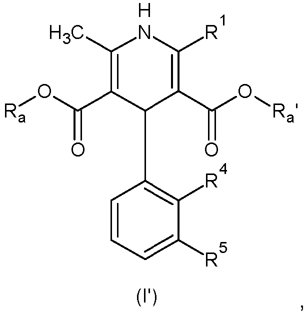 worin R¹, Ra, Ra', R⁴ und R⁵ wie in Anspruch 1 definiert sind.

3. Verbindung zur Verwendung nach Anspruch 1 oder 2, wobei die Verbindung eine Verbindung der Formel (I) ist, in der R² und R³ jeweils unabhängig voneinander eine Estergruppe der Formel -C(O)O-Ra oder der Formel -C(O)O-Ra' sind, oder eine Verbindung der Formel (I'),
worin Ra und Ra' unabhängig voneinander ausgewählt sind aus der Gruppe bestehend aus:
- eine C1-C25-Alkylgruppe Ra1, die gegebenenfalls mindestens ein Heteroatom, ausgewählt aus Stickstoff, Sauerstoff und Schwefel, enthaltend, ausgewählt aus Stickstoff, Sauerstoff und Schwefel, umfasst, wobei die Alkylgruppe Ra1 gegebenenfalls durch mindestens eine Phenylgruppe substituiert ist, wobei die Phenylgruppe gegebenenfalls durch ein Halogenatom substituiert ist,
- eine C1-C25-Alkylgruppe Ra2, die mindestens einen Heterocyclus umfasst, wobei die Alkylgruppe Ra2 gegebenenfalls durch mindestens eine Phenylgruppe substituiert ist, wobei die Phenylgruppe gegebenenfalls durch ein Halogenatom substituiert ist, und
- einen Heterocyclus Ra3, der gegebenenfalls durch mindestens eine C1-C10-Alkylgruppe substituiert ist, die gegebenenfalls mindestens ein Heteroatom, ausgewählt aus Stickstoff, Sauerstoff und Schwefel, enthaltend, ausgewählt aus Stickstoff, Sauerstoff und Schwefel, umfasst und/oder gegebenenfalls durch mindestens eine endständige Phenylgruppe substituiert ist.

4. Verbindung nach einem der Ansprüche 1 bis 3, wobei es sich bei der Verbindung um eine Verbindung der Formel (I) oder (I') handelt, in der R⁴ und R⁵ unabhängig voneinander ausgewählt sind aus der Gruppe bestehend aus:
- ein Wasserstoffatom
- ein Halogenatom,
- eine Hydroxylgruppe,
- eine lineare, zyklische oder verzweigte, gesättigte oder ungesättigte C1-C20-Alkylgruppe, die gegebenenfalls mindestens ein Heteroatom, ausgewählt aus Stickstoff, Sauerstoff und Schwefel, enthaltend, ausgewählt aus Stickstoff, Sauerstoff und Schwefel, enthaltend und/oder gegebenenfalls durch ein Halogenatom substituiert ist,
- eine Carboxylgruppe (-COOH),
- eine Nitrogruppe (-NO₂), und
- eine Cyanogruppe (-CN),
- oder R⁴ und R⁵ bilden zusammen einen Heterocyclus.

5. Verbindung zur Verwendung nach einem der Ansprüche 1 bis 4, wobei die Verbindung eine Verbindung der Formel (I) oder (I') ist, wobei R¹ ausgewählt ist aus der Gruppe bestehend aus einer Methylgruppe CH₃ und einer Aminogruppe NH₂, vorzugsweise ist R¹ eine Methylgruppe.

6. Verbindung zur Verwendung nach einem der Ansprüche 1 bis 5, wobei die Verbindung eine Verbindung der Formel (I) ist, worin R² und R³ jeweils unabhängig voneinander eine Estergruppe der Formel -C(O)O-Ra oder der Formel -C(O)O-Ra' sind, oder eine Verbindung der Formel (I')
worin Ra und Ra' unabhängig voneinander ausgewählt sind aus der Gruppe bestehend aus:
- eine C1-C10-Alkylgruppe, die gegebenenfalls mindestens ein Sauerstoffatom enthaltend,
- eine C1-C25-Alkylgruppe, die mit einer oder zwei endständigen Phenylgruppen substituiert ist und gegebenenfalls mindestens ein Stickstoffatom enthaltend,
- eine C1-C15-Alkylgruppe, die mindestens einen gesättigten Heterocyclus mit 2 bis 7 Kohlenstoffatomen und mindestens einem Stickstoffatom umfasst, wobei der gesättigte Heterocyclus durch mindestens eine Phenylgruppe und/oder durch mindestens eine C1-C10-Alkylgruppe substituiert ist, die gegebenenfalls durch eine oder zwei endständige Phenylgruppen substituiert ist, und
- einen gesättigten Heterocyclus mit 2 bis 7 Kohlenstoffatomen und mindestens einem Stickstoffatom, wobei der Heterocyclus durch mindestens eine Phenylgruppe und/oder durch mindestens eine C1-C10-Alkylgruppe, die gegebenenfalls durch eine oder zwei endständige Phenylgruppen substituiert ist, substituiert ist.

7. Verbindung zur Verwendung nach einem der Ansprüche 1 bis 6, wobei die Verbindung eine Verbindung der Formel (I) oder (I') ist, wobei R₄ Wasserstoff ist und R₅ von Wasserstoff verschieden ist, oder R₅ Wasserstoff ist und R₄ von Wasserstoff verschieden ist.

8. Verbindung zur Verwendung nach einem der Ansprüche 1 bis 7, wobei die Verbindung eine Verbindung der Formel (I) ist, worin R² und R³ jeweils unabhängig voneinander eine Estergruppe der Formel -C(O)O-Ra oder der Formel -C(O)O-Ra' sind, oder eine Verbindung der Formel (I'),
worin Ra und Ra' unabhängig voneinander ausgewählt sind aus der Gruppe bestehend aus:
- eine lineare oder verzweigte, gesättigte C1-C5-Alkylgruppe, die gegebenenfalls mindestens ein Sauerstoffatom enthaltend,
- eine lineare, gesättigte oder ungesättigte C1-C10-Alkylgruppe, substituiert durch eine oder zwei endständige Phenylgruppe(n)
- eine lineare oder verzweigte, gesättigte C1-C5-Alkylgruppe, die durch eine oder zwei endständige Phenylgruppe(n) substituiert ist und mindestens ein Stickstoffatom enthaltend,
- eine C1-C10-Alkylgruppe, die mindestens einen gesättigten Heterocyclus mit 3 bis 5 Kohlenstoffatomen und einem oder zwei Stickstoffatomen umfasst, wobei der gesättigte Heterocyclus mit -CHPh₂ oder -CH₂Ph substituiert ist, und
- einen gesättigten Heterocyclus mit 3 bis 5 Kohlenstoffatomen und einem oder zwei Stickstoffatomen, wobei der gesättigte Heterocyclus durch -CHPh₂ oder - CH₂Ph substituiert ist.

9. Verbindung zur Verwendung nach einem der Ansprüche 1 bis 8, wobei die Verbindung eine Verbindung der Formel (I) oder (I') ist, wobei R⁴ und R⁵ unabhängig voneinander aus der Gruppe ausgewählt sind, die besteht aus:
- ein Wasserstoffatom,
- ein Halogenatom,
- eine lineare oder verzweigte, gesättigte oder ungesättigte C1-C20-Alkylgruppe, die mindestens eine Esterfunktion aufweist, und
- eine Nitrogruppe (-NO₂).

10. Verbindung zur Verwendung nach einem der Ansprüche 1 bis 9, wobei die Verbindung ausgewählt ist aus der Gruppe bestehend aus: 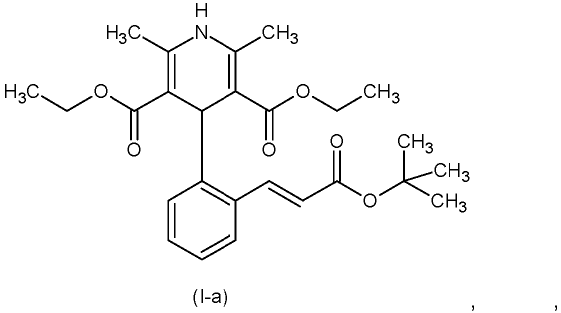 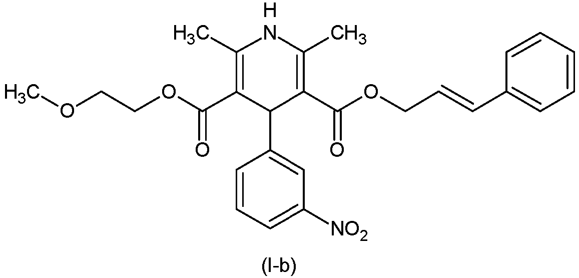 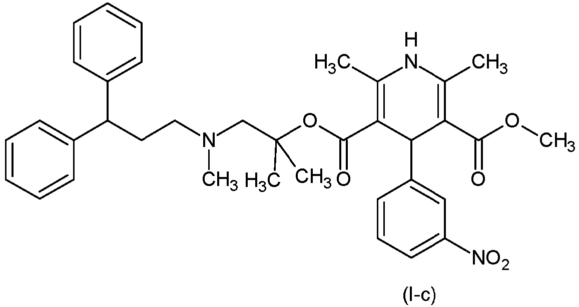 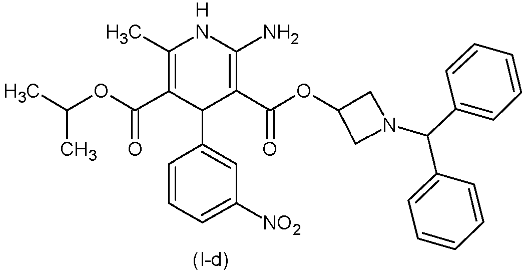 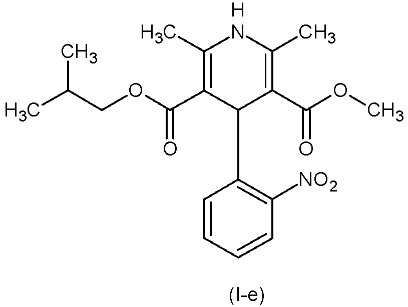 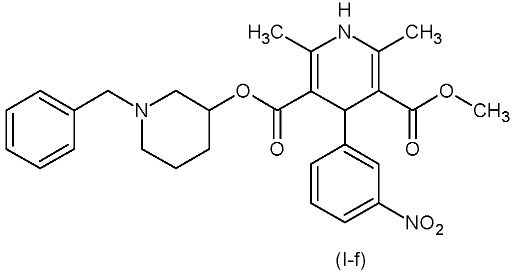 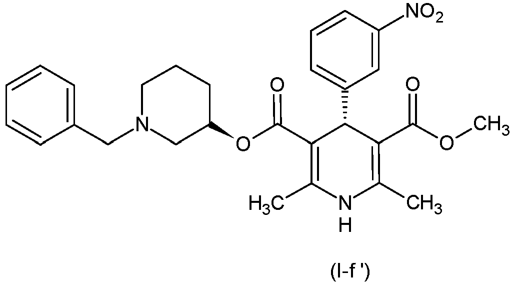 ,und

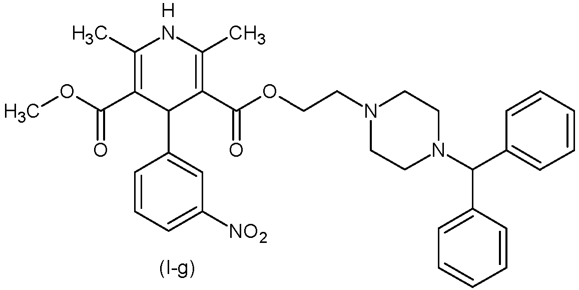

11. Verbindung der Formel (II) zur Verwendung in einem Verfahren zur Behandlung des menschlichen Immunschwächevirus (HIV-1) vom Typ 1, wobei die Formel (II) wie folgt lautet 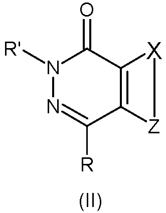
worin X und Z zusammen einen kondensierten Benzolring darstellen, der gegebenenfalls mit mindestens einem Substituenten aus der Gruppe bestehend aus:
- ein Wasserstoffatom,
- ein Halogenatom,
- eine Hydroxylgruppe,
- eine Carboxylgruppe (-COOH),
- eine Nitrogruppe (-NO2) ,
- eine Cyanogruppe (-CN),
- eine lineare oder cyclische oder verzweigte, gesättigte oder ungesättigte C1-C20-Alkylgruppe, die gegebenenfalls substituiert ist und/oder mindestens ein Heteroatom enthaltend, und
- eine Arylgruppe, gegebenenfalls substituiert,
worin R und R' unabhängig voneinander ausgewählt sind aus der Gruppe bestehend aus:
- -ein Wasserstoffatom,
- -ein Halogenatom,
- eine Hydroxylgruppe,
- eine Carboxylgruppe (-COOH),
- eine Nitrogruppe (-NO2) ,
- eine Cyanogruppe (-CN),
- eine Aminogruppe (-NH2) ,
- eine lineare oder zyklische oder verzweigte, gesättigte oder ungesättigte C1-C20-Alkylgruppe, die gegebenenfalls ein Heteroatom und/oder gegebenenfalls mindestens eine innere oder endständige Arylgruppe umfasst, wobei die Arylgruppe gegebenenfalls mit mindestens einem Substituenten substituiert ist, der aus der Gruppe ausgewählt ist, die aus einem Halogenatom, einer Hydroxylgruppe, einer Carboxylgruppe, einer Nitrogruppe, einer Cyanogruppe und einer C1-C10-Alkylgruppe besteht,
- eine C1-C20-Alkylgruppe, die mindestens einen inneren oder endständigen Heterocyclus umfasst, wobei der Heterocyclus gegebenenfalls durch mindestens eine C1-C10-Alkylgruppe substituiert ist,
- eine Arylgruppe, die gegebenenfalls mit mindestens einem Substituenten aus der Gruppe bestehend aus einem Halogenatom, einer Hydroxylgruppe, einer C1-C20-Alkylgruppe, die gegebenenfalls ein Halogenatom und/oder ein Heteroatom umfasst, Carboxylgruppe, einer Nitrogruppe und Cyanogruppe substituiert ist, und
- einen Heterocyclus, der gegebenenfalls durch mindestens eine C1-C10-Alkylgruppe substituiert ist,
und/oder ein Salz davon und/oder ein Solvat davon.

12. Verbindung zur Verwendung nach Anspruch 11, wobei die Verbindung eine Verbindung der Formel (II') wie folgt ist 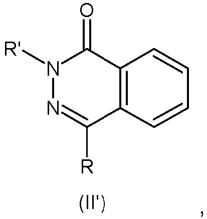 worin R und R' wie in Anspruch 11 definiert sind.

13. Verbindung zur Verwendung nach Anspruch 11 oder 12, wobei die Verbindung eine Verbindung der Formel (II) oder (II') ist, wobei R ausgewählt ist aus der Gruppe bestehend aus
- eine lineare oder zyklische oder verzweigte, gesättigte oder ungesättigte C1-C20-Alkylgruppe, die gegebenenfalls ein Heteroatom und/oder gegebenenfalls mindestens eine innere oder endständige Arylgruppe umfasst, wobei die Arylgruppe gegebenenfalls mit mindestens einem Substituenten substituiert ist, der aus der Gruppe ausgewählt ist, die aus einem Halogenatom, einer Hydroxylgruppe, einer Carboxylgruppe, einer Nitrogruppe, einer Cyanogruppe und einer C1-C20-Alkylgruppe besteht, und
- eine Arylgruppe, die gegebenenfalls mit mindestens einem Substituenten aus der Gruppe bestehend aus einem Halogenatom, einer Hydroxylgruppe, einer C1-C20-Alkylgruppe, die gegebenenfalls ein Halogenatom und/oder ein Heteroatom umfasst, einer Carboxylgruppe, einer Nitrogruppe und einer Cyanogruppe substituiert ist.

14. Verbindung zur Verwendung nach einem der Ansprüche 11, 12 oder 13, wobei die Verbindung eine Verbindung der Formel (II) oder (II') ist, worin R' ausgewählt ist aus der Gruppe bestehend aus:
- eine C1-C20-Alkylgruppe, die mindestens einen inneren oder endständigen gesättigten Heterocyclus mit 2 bis 7 Kohlenstoffatomen und mindestens einem Stickstoffatom umfasst, wobei der Heterocyclus gegebenenfalls durch mindestens eine C1-C10-Alkylgruppe substituiert ist, und
- einen gesättigten Heterocyclus mit 2 bis 7 Kohlenstoffatomen und mindestens einem Stickstoffatom, der gegebenenfalls durch mindestens eine C1-C10-Alkylgruppe substituiert ist.

15. Verbindung zur Verwendung nach einem der Ansprüche 11 bis 14, wobei die Verbindung eine Verbindung der Formel (II) oder (II') ist, worin R eine - CH₂-Ph-Gruppe ist, wobei Ph eine Arylgruppe ist, die gegebenenfalls mit mindestens einem Substituenten substituiert ist, der aus der Gruppe ausgewählt ist, die aus einem Halogenatom, einer Hydroxylgruppe, einer C1-C20-Alkylgruppe, einer Carboxylgruppe, einer Nitrogruppe und einer Cyanogruppe besteht.

16. Verbindung zur Verwendung nach einem der Ansprüche 11 bis 15, wobei die Verbindung eine Verbindung der Formel (II) oder (II') ist, wobei R' ein gesättigter Heterocyclus ist, der 2 bis 7 Kohlenstoffatome und mindestens ein Stickstoffatom umfasst, wobei der Heterocyclus gegebenenfalls durch mindestens eine C1-C10-Alkylgruppe substituiert ist.

17. Verbindung zur Verwendung nach einem der Ansprüche 11 bis 16, wobei die Verbindung die folgende Struktur aufweist: .

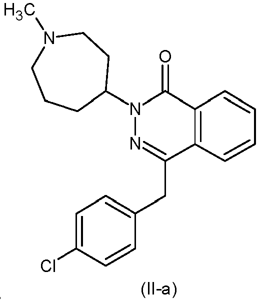

18. Verbindung zur Verwendung nach einem der Ansprüche 1 bis 17, wobei die Behandlung des menschlichen Immundefizienzvirus vom Typ 1 die Behandlung des erworbenen Immunschwächesyndroms ist.

19. Pharmazeutische Zusammensetzung oder Kit, umfassend eine Verbindung der Formel (I) oder (I') und/oder (II) oder (II') nach einem der Ansprüche 1 bis 17 zur Verwendung in einem Verfahren zur Behandlung eines menschlichen Immunschwächevirus (HIV-1) vom Typ 1.

## Revendications

1. Composé de formule (I) pour son utilisation dans une méthode de traitement du virus de l'immunodéficience humaine de type 1 (VIH-1), dans lequel ladite formule (I) est la suivante : 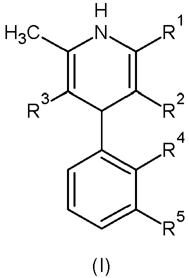
dans lequel R¹est choisi dans le groupe consistant en :
- un atome d'hydrogène,
- un groupe amino NH₂, et
- un groupe alkyle en C1-C10, linéaire, cyclique ou ramifié, saturé ou insaturé, comprenant éventuellement un hétéroatome choisi parmi l'azote, l'oxygène et le soufre,
dans lequel R², R³, R⁴et R⁵sont indépendamment choisis dans le groupe consistant en :
- un atome d'hydrogène,
- un atome d'halogène,
- un groupe hydroxyle,
- un groupe alkyle en C1-C20, linéaire, cyclique ou ramifié, saturé ou insaturé, éventuellement substitué et/ou comprenant éventuellement un hétéroatome,
- un groupe aryle, éventuellement substitué par au moins un groupe alkyle en C1-C20, linéaire ou cyclique ou ramifié, saturé ou insaturé, éventuellement substitué et/ou comprenant éventuellement au moins un hétéroatome,
- un groupe ester de formule -C(O)O-Rₐou de formule -C(O)O-R_{(a)'}, Rₐet Rₐ' étant choisis indépendamment dans le groupe constitué d'un groupe alkyle en C1-C30, linéaire ou cyclique ou ramifié, saturé ou insaturé, éventuellement substitué et/ou comprenant éventuellement au moins un hétéroatome, ou Rₐétant un groupe alkylaryle en C1-C30, dans lequel l'alkyle est linéaire ou cyclique ou ramifié, saturé ou insaturé, lié au groupe aryle, éventuellement substitué et/ou comprenant éventuellement au moins un hétéroatome :
- un groupe carboxyle (-COOH),
- un groupe nitro (-NO₂) , et
- un groupe cyano (-CN),
dans lequel R⁴et R₅forment éventuellement ensemble un hétérocycle,
et/ou un de ses sels et/ou un de ses solvants.

2. Composé pour son utilisation selon la revendication 1, dans lequel ledit composé est un composé de formule (I') comme suit : 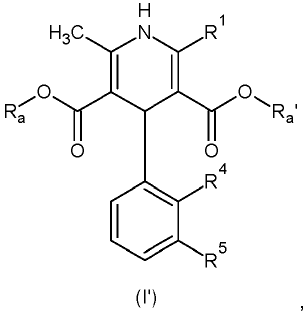 dans lequel R¹, Rₐ, R_{(a)'}, R⁴et R⁵sont tels que définis dans la revendication 1.

3. Composé pour son utilisation selon la revendication 1 ou 2, dans lequel ledit composé est un composé de formule (I) dans lequel R²et R³sont chacun indépendamment un groupe ester de formule -C(O)O-Rₐou de formule -C(O)O-R_{(a)'}, ou un composé de formule (I'),
dans lequel Rₐet Rₐ' sont indépendamment choisis dans le groupe consistant en :
- un groupe alkyle en C1-C25 Rₐ₁comprenant éventuellement au moins un hétéroatome choisi parmi l'azote, l'oxygène et le soufre, ledit groupe alkyle Rₐ₁étant éventuellement substitué par au moins un groupe phényle, ledit groupe phényle étant éventuellement substitué par un atome d'halogène,
- un groupe alkyle R_{(a)'(2)}en C1-C25 comprenant au moins un hétérocycle, ledit groupe alkyle R_{(a)'(2)}étant éventuellement substitué par au moins un groupe phényle, ledit groupe phényle étant éventuellement substitué par un atome d'halogène, et
- un hétérocycle R_{(a)'(3)}éventuellement substitué par au moins un groupe alkyle en C1-10 comprenant éventuellement au moins un hétéroatome choisi parmi l'azote, l'oxygène et le soufre et/ou éventuellement substitué par au moins un groupe phényle terminal.

4. Composé selon l'une quelconque des revendications 1 à 3, dans lequel ledit composé est un composé de formule (I) ou (I'), dans lequel R⁴et R⁵sont indépendamment choisis dans le groupe constitué de :
- un atome d'hydrogène
- un atome d'halogène,
- un groupe hydroxyle,
- un groupe alkyle en C1-C20, linéaire ou cyclique ou ramifié, saturé ou insaturé, comprenant éventuellement au moins un hétéroatome choisi parmi l'azote, l'oxygène et le soufre et/ou éventuellement substitué par un atome d'halogène,
- un groupe carboxyle (-COOH),
- un groupe nitro (-NO₂) , et
- un groupe cyano (-CN),
ou R⁴et R₅forment ensemble un hétérocycle.

5. Composé pour son utilisation selon l'une quelconque des revendications 1 à 4, dans lequel ledit composé est un composé de formule (I) ou (I'), dans lequel R¹est choisi dans le groupe constitué d'un groupe méthyle CH₃et d'un groupe amino NH_{(2) (,)}de préférence R¹est un groupe méthyle.

6. Composé pour son utilisation selon l'une quelconque des revendications 1 à 5, dans lequel ledit composé est un composé de formule (I) dans lequel R²et R³sont chacun indépendamment un groupe ester de formule -C(O)O-Rₐou de formule - C(O)O-R_{(a)'}, ou un composé de formule (I')
dans lequel Rₐet Rₐ' sont indépendamment choisis dans le groupe consistant en :
- un groupe alkyle de C1-C10 comprenant éventuellement au moins un atome d'oxygène,
- un groupe alkyle en C1-C25 substitué par un ou deux groupes phényles terminaux et comprenant éventuellement au moins un atome d'azote
- un groupe alkyle en C1-C15 comprenant au moins un hétérocycle saturé comprenant de 2 à 7 atomes de carbone et au moins un atome d'azote, ledit hétérocycle saturé étant substitué par au moins un groupe phényle et/ou par au moins un groupe alkyle en C1-C10 éventuellement substitué par un ou deux groupes phényle terminaux, et
- un hétérocycle saturé comprenant de 2 à 7 atomes de carbone et au moins un atome d'azote, ledit hétérocycle étant substitué par au moins un groupe phényle et/ou par au moins un groupe alkyle en C1-C10 éventuellement substitué par un ou deux groupes phényles terminaux.

7. Composé pour son utilisation selon l'une des revendications 1 à 6, dans lequel ledit composé est un composé de formule (I) ou (I'), dans lequel R₄est de l'hydrogène et R₅est différent de l'hydrogène, ou R₅est de l'hydrogène et R₄est différent de l'hydrogène.

8. Composé pour son utilisation selon l'une quelconque des revendications 1 à 7, dans lequel ledit composé est un composé de formule (I) dans lequel R²et R³sont chacun indépendamment un groupe ester de formule -C(O)O-Rₐou de formule - C(O)O-R_{(a)'}, ou un composé de formule (I'),
dans lequel Rₐet Rₐ' sont indépendamment choisis dans le groupe consistant en :
- un groupe alkyle en C1-C5, linéaire ou ramifié, saturé, comprenant éventuellement au moins un atome d'oxygène,
- un groupe alkyle en C1-C10, linéaire, saturé ou insaturé, substitué par un ou deux groupes phényles terminaux
- un groupe alkyle en C1-C5, linéaire ou ramifié, saturé, substitué par un ou deux groupes phényles terminaux et comprenant au moins un atome d'azote,
- un groupe alkyle en C1-C10, comprenant au moins un hétérocycle saturé comprenant de 3 à 5 atomes de carbone et un ou deux atomes d'azote, ledit hétérocycle saturé étant substitué par -CHPh₂ou -CH₂Ph, et
- un hétérocycle saturé comprenant de 3 à 5 atomes de carbone et un ou deux atomes d'azote, ledit hétérocycle saturé étant substitué par -CHPh₂ou -CH₂Ph.

9. Composé pour son utilisation selon l'une quelconque des revendications 1 à 8, dans lequel ledit composé est un composé de formule (I) ou (I'), dans lequel R⁴et R⁵sont indépendamment choisis dans le groupe constitué par :
- un atome d'hydrogène,
- un atome d'halogène,
- un groupe alkyle en C1-C20, linéaire ou ramifié, saturé ou insaturé, comprenant au moins une fonction ester, et
- un groupe nitro (-NO₂₎.

10. Composé pour son utilisation selon l'une des revendications 1 à 9, dans lequel ledit composé est choisi dans le groupe constitué par : 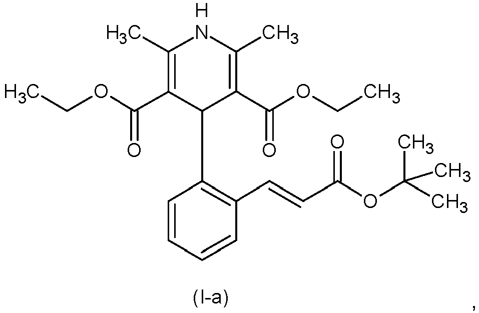 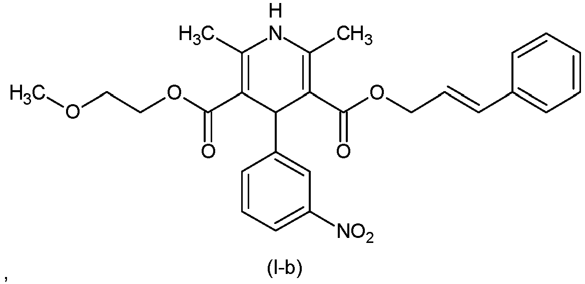 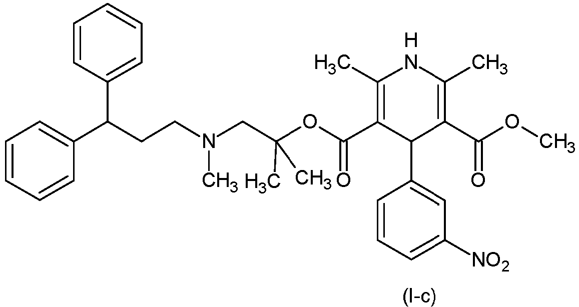 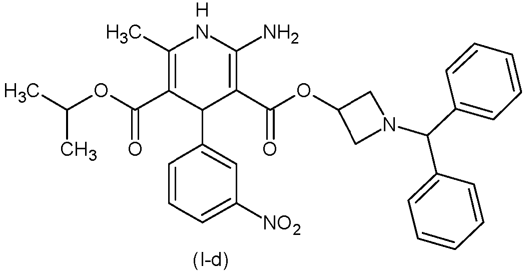 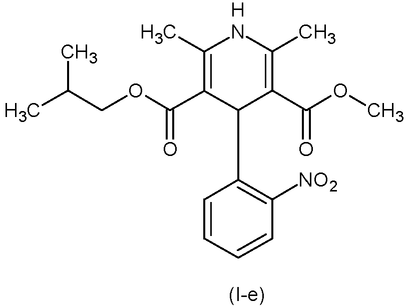 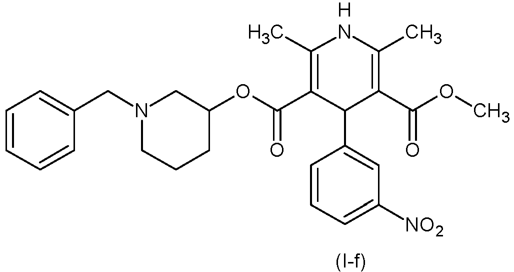 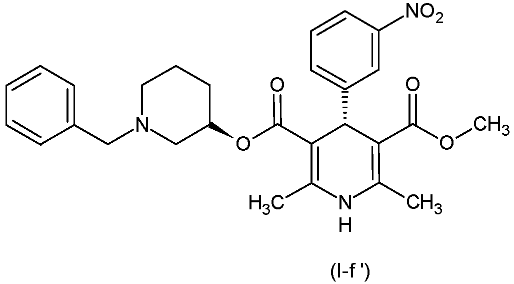 ,et

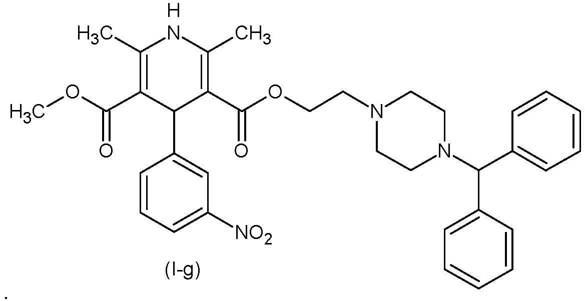

11. Composé de formule (II) pour son utilisation dans une méthode de traitement du virus de l'immunodéficience humaine de type 1 (VIH-1), dans lequel ladite formule (II) est la suivante 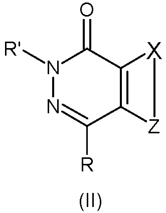
dans lequel X et Z représentent ensemble un anneau benzénique fusionné, éventuellement substitué par au moins un substituant choisi dans le groupe constitué par :
- un atome d'hydrogène,
- un atome d'halogène,
- un groupe hydroxyle,
- un groupe carboxyle (-COOH),
- un groupe nitro (-NO₂) ,
- un groupe cyano (-CN),
- un groupe alkyle en C1-C20, linéaire, cyclique ou ramifié, saturé ou insaturé, éventuellement substitué et/ou comprenant éventuellement au moins un hétéroatome, et
- un groupe aryle, éventuellement substitué,
dans lequel R et R' sont indépendamment choisis dans le groupe consistant en :
- un atome d'hydrogène,
- un atome d'halogène,
- un groupe hydroxyle,
- un groupe carboxyle (-COOH),
- un groupe nitro (-NO₂) ,
- un groupe cyano (-CN),
- un groupe amino (-NH₂₎,
- un groupe alkyle en C1-C20, linéaire, cyclique ou ramifié, saturé ou insaturé, comprenant éventuellement un hétéroatome et/ou comprenant éventuellement au moins un groupe aryle interne ou terminal, ledit groupe aryle étant éventuellement substitué par au moins un substituant choisi dans le groupe constitué par un atome d'halogène, un groupe hydroxyle, un groupe carboxyle, un groupe nitro, un groupe cyano et un groupe alkyle en C1-C10,
- un groupe alkyle en C1-C20 comprenant au moins un hétérocycle interne ou terminal, ledit hétérocycle étant éventuellement substitué par au moins un groupe alkyle en C1-C10,
- un groupe aryle, éventuellement substitué par au moins un substituant choisi dans le groupe constitué d'un atome d'halogène, d'un groupe hydroxyle, d'un groupe alkyle de C1-C20 comprenant éventuellement un atome d'halogène et/ou un hétéroatome, d'groupe carboxyle, d'un groupe nitro et d'un groupe cyano, et
- un hétérocycle éventuellement substitué par au moins un groupe alkyle en C1-C10,
et/ou un de ses sels et/ou un de ses solvants.

12. Composé pour son utilisation selon la revendication 11, dans lequel ledit composé est un composé de formule (II') comme suit 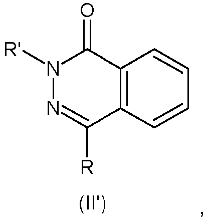 dans lequel R et R' sont tels que définis dans la revendication 11.

13. Composé pour son utilisation selon la revendication 11 ou 12, dans lequel ledit composé est un composé de formule (II) ou (II'), dans lequel R est choisi dans le groupe constitué par
- un groupe alkyle en C1-C20, linéaire, cyclique ou ramifié, saturé ou insaturé, comprenant éventuellement un hétéroatome et/ou comprenant éventuellement au moins un groupe aryle interne ou terminal, ledit groupe aryle étant éventuellement substitué par au moins un substituant choisi dans le groupe constitué par un atome d'halogène, un groupe hydroxyle, un groupe carboxyle, un groupe nitro, un groupe cyano et un groupe alkyle en C1-C20, et
- un groupe aryle, éventuellement substitué par au moins un substituant choisi dans le groupe constitué d'un atome d'halogène, d'un groupe hydroxyle, d'un groupe alkyle de C1-C20 comprenant éventuellement un atome d'halogène et/ou un hétéroatome, d'un groupe carboxyle, d'un groupe nitro et d'un groupe cyano.

14. Composé pour son utilisation selon l'une quelconque des revendications 11, 12 ou 13, dans lequel ledit composé est un composé de formule (II) ou (II'), dans lequel R' est choisi dans le groupe constitué par :
- un groupe alkyle en C1-C20 comprenant au moins un hétérocycle saturé interne ou terminal comprenant de 2 à 7 atomes de carbone et au moins un atome d'azote, ledit hétérocycle étant éventuellement substitué par au moins un groupe alkyle en C1-C10, et
- un hétérocycle saturé comprenant de 2 à 7 atomes de carbone et au moins un atome d'azote, éventuellement substitué par au moins un groupe alkyle en C1-C10.

15. Composé pour son utilisation selon l'une quelconque des revendications 11 à 14, dans lequel ledit composé est un composé de formule (II) ou (II'), où R est un groupe -CH₂-Ph, Ph étant un groupe aryle, éventuellement substitué par au moins un substituant choisi dans le groupe constitué par un atome d'halogène, un groupe hydroxyle, un groupe alkyle en C1-C20, un groupe carboxyle, un groupe nitro et un groupe cyano.

16. Composé pour son utilisation selon l'une quelconque des revendications 11 à 15, dans lequel ledit composé est un composé de formule (II) ou (II'), où R' est un hétérocycle saturé comprenant de 2 à 7 atomes de carbone et au moins un atome d'azote, ledit hétérocycle étant éventuellement substitué par au moins un groupe alkyle en C1-C10.

17. Composé pour son utilisation selon l'une quelconque des revendications 11 à 16, dans lequel ledit composé présente la structure suivante:

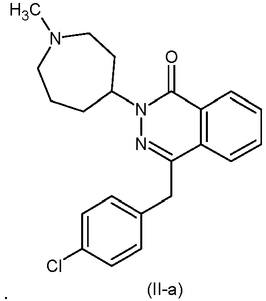

18. Composé pour son utilisation selon l'une des revendications 1 à 17, dans lequel le traitement du virus de l'immunodéficience humaine de type 1 est le traitement du syndrome d'immunodéficience acquise.

19. Composition pharmaceutique ou kit comprenant un composé de formule (I) ou (l') et/ou (II) ou (II') selon l'une quelconque des revendications 1 à 17 pour son utilisation dans une méthode de traitement du virus de l'immunodéficience humaine de type 1 (VIH-1).
